# EUROPEAN PATENT APPLICATION

(11) **EP 4 769 330 A1**
(43) Date of publication of application: **01.07.2026**
(21) Application number: 25227911.2
(22) Date of filing: 31.12.2025
(51) Int. Cl.: G06V 10/44, G06T 7/00, G06V 10/75, G06V 10/764, G06V 10/82, G06V 10/98, G06V 20/40, G06V 10/26

(54) **METHODS AND SYSTEMS FOR IMAGE PROCESSING**

(30) Priority: 31.12.2024 CN 202411999467; 31.12.2024 CN 202412000053
(71) Applicant: Shanghai United Imaging Healthcare Co., Ltd., Shanghai 201807 (CN)
(72) Inventor: WANG, Xiaodong, Shanghai, 201807 (CN); LI, Yihao, Shanghai, 201807 (CN); RUAN, Shijian, Shanghai, 201807 (CN)
(74) Representative: Wang, Bo

(57) **Abstract**

Disclosed is a method for image processing. The method includes: obtaining one or more image sequences, each of the one or more image sequences including one or more images; determining key information of at least a portion of the one or more images in each of the one or more image sequences; and processing the one or more image sequences based on feature information of each of the one or more image sequences, the feature information of each of the one or more image sequences including the key information of the at least a portion of the one or more images in the image sequence.

## Description

### CROSS REFERENCE TO RELATED APPLICATIONS

This application claims the priority to Chinese patent application No. 202412000053.X filed on December 31, 2024, and Chinese patent application No. 202411999467.1 filed on December 31, 2024, the entire content of which are incorporated herein by reference.

### TECHNICAL FIELD

The present disclosure generally relates to a field of image processing, and in particular to a method and system for image processing.

### BACKGROUND

In the field of imaging, with a development of artificial intelligence (AI) technology and a rapid advancement of imaging devices, image processing technologies such as an image recognition, an image segmentation, and an advanced analysis based on image content are widely applied in various clinical scenarios. These technologies cover an entire clinical workflow, including diagnosis, treatment planning, surgical planning, and follow-up, and provide an auxiliary support for clinicians. In some application scenarios, a joint analysis of a plurality of images also significantly improves a diagnostic accuracy. However, if an image is input into an incorrect application scenario for use, an expected image display or processing result may not be achieved, thereby affecting a diagnostic judgment of a physician.

In clinical practice, a user (e.g., the physician) often needs to display the images according to specific clinical requirements, or compare and display the images with different requirements in the same interface to assist the physician in making more accurate diagnosis and analysis. Furthermore, before reading images, the physician also needs to perform a large amount of pre-processing work, including a transmission of the images, a sequence selection, an application of post-processing algorithms, etc. These steps are cumbersome and time-consuming to perform manually and rely on professional knowledges and experience of the physician, which further increase a complexity of the workflow.

Based on this, it is desirable to propose a method to provide the user with required images and image application scenarios, thereby reducing the complexity of the work of the user, and improving the work efficiency and diagnostic accuracy of the user.

### SUMMARY

One or more embodiments of the present disclosure provide a method for image processing. The method for image processing includes: obtaining one or more image sequences, each of the one or more image sequences including one or more images; determining key information of at least a portion of the one or more images in each of the one or more image sequences, the key information at least including at least one of anatomical region information, disease information, image quality information, image modality information, an image sequence type, or contrast information; and processing the one or more image sequences based on feature information of each of the one or more image sequences, the feature information of each of the one or more image sequences including the key information of the at least a portion of the one or more images in the image sequence.

In some embodiments, the one or more image sequences includes a first image sequence and one or more second image sequences, wherein the first image sequence has a largest axial field of view among the one or more image sequences, the first image sequence includes one or more first images, and each of the one or more second image sequences includes one or more second images, the determining key information of at least a portion of the one or more images in each of the one or more image sequences includes: determining key information of each of at least a portion of the one or more first images in the first image sequence; and for each of at least a portion of the one or more second images in each of the one or more second image sequences, determining a matching result between the second image and each of the at least a portion of the one or more first images in the first image sequence; determining the key information of the second image based on the matching result and the key information of each of the at least a portion of the one or more first images in the first image sequence.

In some embodiments, determining a matching result between the second image and the at least a portion of the one or more first images in the first image sequence includes: determining a matching result between a second position of the second image and a first position of each of the at least a portion of the one or more first images in the first image sequence.

In some embodiments, the determining the key information of the second image based on the matching result and the key information of each of the at least a portion of the one or more first images in the first image sequence includes: in response to the matching result being that the second position of the second image is consistent with the first position of the first image, determining the key information of the first image corresponding to the matching result as the key information of the second image corresponding to the matching result; in response to the matching result being that the second position of the second image is not consistent with the first position of the first image, determining the key information of the second image by processing the second image through a key information extraction model.

In some embodiments, the processing the one or more image sequences based on feature information of each of the one or more image sequences includes: determining one or more target image sequences from the one or more image sequences based on the key information of the at least a portion of the one or more images in each of the one or more image sequences; sending the one or more target image sequences to a user terminal for display.

In some embodiments, the feature information of each of the one or more image sequences further includes a sequence label, the determining one or more target image sequences from the one or more image sequences based on the key information of the at least a portion of the one or more images in each of the one or more image sequences includes: determining the sequence label of each of the one or more image sequences based on the key information of the at least a portion of the one or more images in each of the one or more image sequences; determining a search label used to determine whether the sequence label of each of the one or more image sequences satisfies at least one search condition; determining the one or more target image sequences from the one or more image sequences based on the search label and the sequence label of each of the one or more image sequences.

In some embodiments, the determining the sequence label of each of the one or more image sequences based on the key information of the at least a portion of the one or more images in each of the one or more image sequences includes: determining a frequency of occurrence of various key information based on the key information of the at least a portion of the one or more images in the image sequence; determining the sequence label of the image sequence based on the frequency of occurrence of the various key information.

In some embodiments, the processing the one or more image sequences based on feature information of each of the one or more image sequences includes: determining at least one target application scenario adapted to the one or more image sequences based on the key information of the at least a portion of images in each of the one or more image sequences.

In some embodiments, the feature information of each of the one or more image sequences further includes auxiliary information of the at least a portion of the one or more images in each of the one or more image sequences, the auxiliary information including metadata of each image and/or clinical data information of an imaging object corresponding to the image sequence, the determining at least one target application scenario adapted to the one or more image sequences includes: based on the key information and the auxiliary information of the at least a portion of images in the one or more image sequences, selecting at least one candidate application scenario adapted to the one or more image sequences from a plurality of candidate application scenarios as the at least one target application scenario.

In some embodiments, the selecting at least one candidate application scenario adapted to the one or more image sequences including: matching the key information and the auxiliary information of images in a set of images with constraint conditions of the plurality of candidate application scenarios, to determine the at least one candidate application scenario adapted to the one or more image sequences from the plurality of candidate application scenarios as at least one target application scenario, wherein the set of images includes the at least a portion of images in each of the one or more image sequences.

In some embodiments, the determining at least one target application scenario adapted to the one or more image sequences further includes: for each image sequence in the one or more image sequences, based on the key information of each image in the at least a portion of the one or more images, performing pre-processing on the image sequence to obtain a pre-processing result corresponding to each image in the at least a portion of the one or more images, wherein the pre-processing includes at least one of image segmentation, image alignment, image classification, and artifact removal; based on the pre-processing result, updating the key information of each image in the at least a portion of the one or more images.

In some embodiments, the processing the one or more image sequences based on feature information of each of the one or more image sequences includes: for each image sequence in the one or more image sequences, based on the key information of each image in the at least a portion of the one or more images in the image sequence, determining a processing strategy for the image; processing each image in the at least a portion of the one or more images in the image sequence based on the processing strategy for the image.

In some embodiments, the feature information of each of the one or more image sequences further includes additional information corresponding to each of the one or more image sequences, the determining key information of at least a portion of the one or more images in each of the one or more image sequences including: extracting an additional feature of the additional information; extracting an image feature of the image; and determining the key information by fusing the image feature and the additional feature.

In some embodiments, the determining a processing strategy for the image sequence includes: in response to the key information of at least one image in the image sequence reflecting that an imaging object corresponding to the image sequence is an emergency case, pushing the at least one image and/or the key information of the at least one image to a user terminal for emergency handling by a physician; in response to the key information of none of the images in the image sequence reflecting that the imaging object is the emergency case, for each image in the image sequence, processing the image through a post-processing algorithm corresponding to the image based on the key information of the image, to obtain a post-processing result and pushing the post-processing result to the user terminal.

One or more embodiments of the present disclosure provide a computer device. The computer device comprising a memory and a processor, wherein the memory stores a computer program, and when the computer program is executed by the processor, the method for image processing is realized.

### BRIEF DESCRIPTION OF THE DRAWINGS

The present disclosure is further described in an illustrative manner by way of exemplary embodiments. These exemplary embodiments are described in detail with reference to the accompanying drawings. These embodiments are not limiting. In these embodiments, the same reference numerals denote the same structure.
FIG. 1 is a schematic diagram illustrating a system for image processing according to some embodiments of the present disclosure;
FIG. 2 is a diagram illustrating an internal structure of a processing device according to some embodiments of the present disclosure;
FIG. 3 is a block diagram illustrating a system for image processing according to some embodiments of the present disclosure;
FIG. 4 is a flowchart illustrating an exemplary method for image processing according to some embodiments of the present disclosure;
FIG. 5 is a model structure diagram illustrating a key information extraction model according to some embodiments of the present disclosure;
FIG. 6 is a schematic diagram illustrating a display layout of one or more target image sequences according to some embodiments of the present disclosure;
FIG. 7 is a flowchart illustrating an exemplary training process of a key information extraction model according to some embodiments of the present disclosure;
FIG. 8 is a flowchart illustrating an exemplary method for determining key information of a second image according to some embodiments of the present disclosure;
FIG. 9 is a flowchart illustrating an exemplary method for determining one or more target image sequences according to some embodiments of the present disclosure;
FIG. 10 is another flowchart illustrating an exemplary method for determining one or more target image sequences according to some embodiments of the present disclosure;
FIG. 11 is another flowchart illustrating an exemplary method for image processing according to some embodiments of the present disclosure.

### DETAILED DESCRIPTION

The illustrate the technical solutions of the embodiments of the present disclosure more clearly, the following briefly introduces the accompanying drawings required for describing the embodiments. Obviously, the accompanying drawings in the following description are merely some examples or embodiments of the present disclosure. For those skilled in the art, the present disclosure may be applied to other similar scenarios based on these drawings without creative efforts. Unless obviously acquired from the context or the context illustrates otherwise, the same numeral in the drawings refers to the same structure or operation.

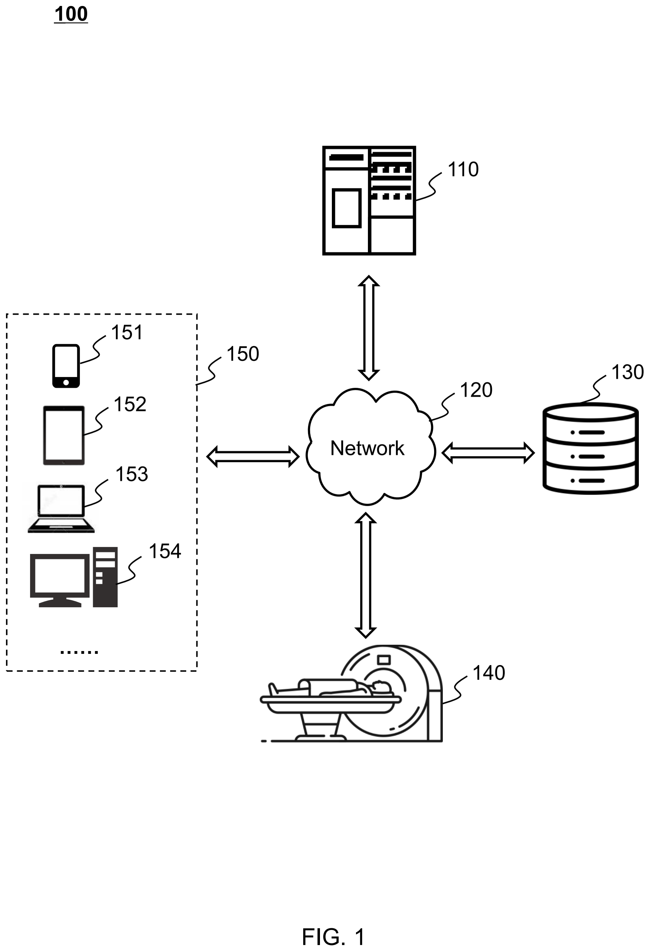

FIG. 1 is a schematic diagram illustrating a system for image processing according to some embodiments of the present disclosure. As shown in FIG. 1, a system for image processing 100 (hereinafter referred to as the system 100) may include a processing device 110, a network 120, a storage device 130, an imaging device 140, and a user terminal 150.

The processing device 110 refers to a device configured to process data and/or information from at least one component of the system 100 or an external data source (e.g., a cloud data center). The processing device 110 may access the data or information from the storage device 130, the imaging device 140, and/or the user terminal 150 via the network 120. The processing device 110 may also be directly connected to the storage device 130, the imaging device 140, and/or the user terminal 150 to access the information and/or data. For example, the processing device 110 acquires images and/or image sequences collected by the imaging device 140 from the imaging device 140. As another example, the processing device 110 determines at least one target application scenario adapted to one or more image sequences based on key information of at least a portion of images in one or more image sequences. More descriptions regarding the one or more image sequences, the key information, and the target application scenario may be found in FIG. 12 and related descriptions.

The network 120 may connect various components of the system 100 and/or connect the system 100 with external resource portions. In some embodiments, one or more components of the system 100 (e.g., the processing device 110, the storage device 130, the imaging device 140, and/or the user terminal 150) exchange the information and/or data via the network 120.

In some embodiments, the network 120 is any one or more of a wired network or a wireless network. In some embodiments, the network 120 includes one or more network access points. For example, the network 120 include wired or wireless network access points (e.g., base stations and/or network switching points). Through these network access points, the one or more components of the system 100 are connected to the network 120 to exchange the data and/or information.

The storage device 130 refers to a device configured to store data, instructions, and/or any other information. In some embodiments, the storage device 130 stores data and/or information acquired from the processing device 110, the imaging device 140, the user terminal 150, etc. For example, the storage device 130 stores images and/or image sequences generated by the imaging device 140. As another example, the storage device 130 stores a plurality of candidate application scenarios. In some embodiments, the storage device 130 includes a mass storage, a removable storage, etc., or any combination thereof.

The imaging device 140 refers to a device capable of imaging an imaging object (e.g., a patient, an experimental subject, etc.). For example, the imaging device 140 is a computed tomography (CT) device, a positron emission computed tomography (PET) device, a magnetic resonance imaging (MRI) device, etc.

The user terminal 150 refers to one or more terminals or software used by a user of the imaging device 140 (e.g., a physician, a researcher, etc., who uses the imaging device 140). In some embodiments, as shown in FIG. 1, the user terminal 150 includes, but is not limited to, a smart phone 151, a tablet computer 152, a laptop computer 153, a desktop computer 154, etc. In some embodiments, the user terminal 150 interacts with other components in the system 100 via the network 120. For example, the user terminal 150 acquires the images or image sequences determined by the imaging device 140 and/or the key information of images determined by the processing device 110 via the network 120, and displays them to the user for viewing. As another example, the user terminal 150 controls a scanning process of the imaging device 140 (e.g., controlling the imaging device 140 to start scanning, etc.) via the network 120.

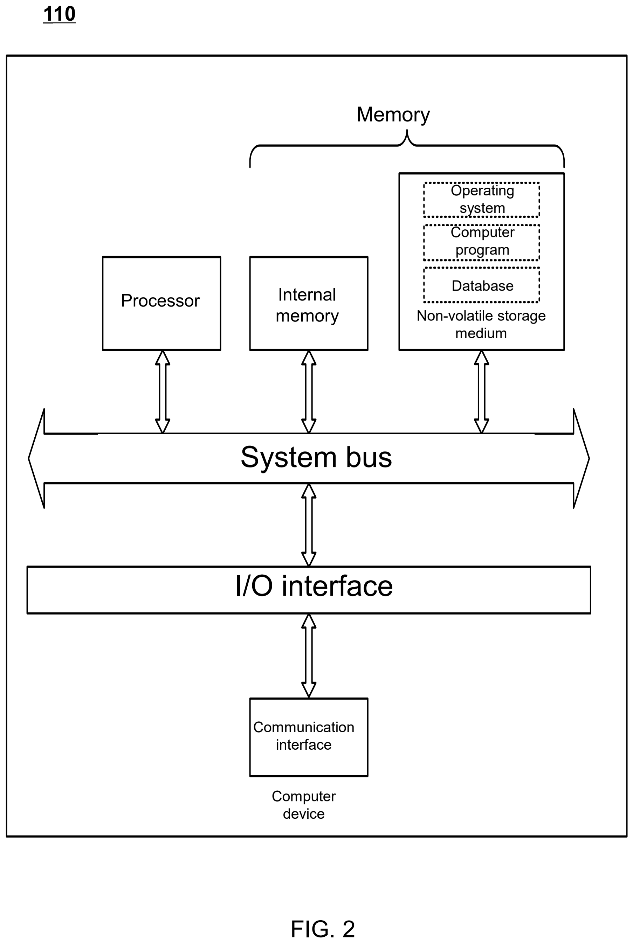

FIG. 2 is a diagram illustrating an internal structure of a computer device according to some embodiments of the present disclosure.

As shown in FIG. 2, the processing device 110 includes a processor, a memory, an input/output (I/O) interface, and a communication interface. The processor, the memory, and the I/O interface are connected via a system bus. The communication interface is connected to the system bus via the I/O interface. The processor is configured to provide computing and control capabilities. The memory includes a non-volatile storage medium and an internal memory. The non-volatile storage medium stores an operating system, computer programs, and a database. The internal memory provides an environment for executions of the operating system and computer programs in the non-volatile storage medium. The database is configured to store data related to image processing. The I/O interface is configured to exchange information between the processor and an external device. The communication interface is configured to connect and communicate with an external terminal (e.g., the user terminal 150) via a network (e.g., the network 120). The computer program, when executed by the processor, implements the method for image processing. A server may be an independent physical server, a server cluster or a distributed system composed of a plurality of physical servers, or a cloud server providing cloud computing services.

It may be understood that the structure shown in FIG. 2 is merely a block diagram of a part of the structure related to the processing device described in the present disclosure, and does not constitute a limitation on the processing device described in the present disclosure. For example, the processing device includes more or fewer components than those shown in FIG. 2, combines some components, or has a different arrangement of components.

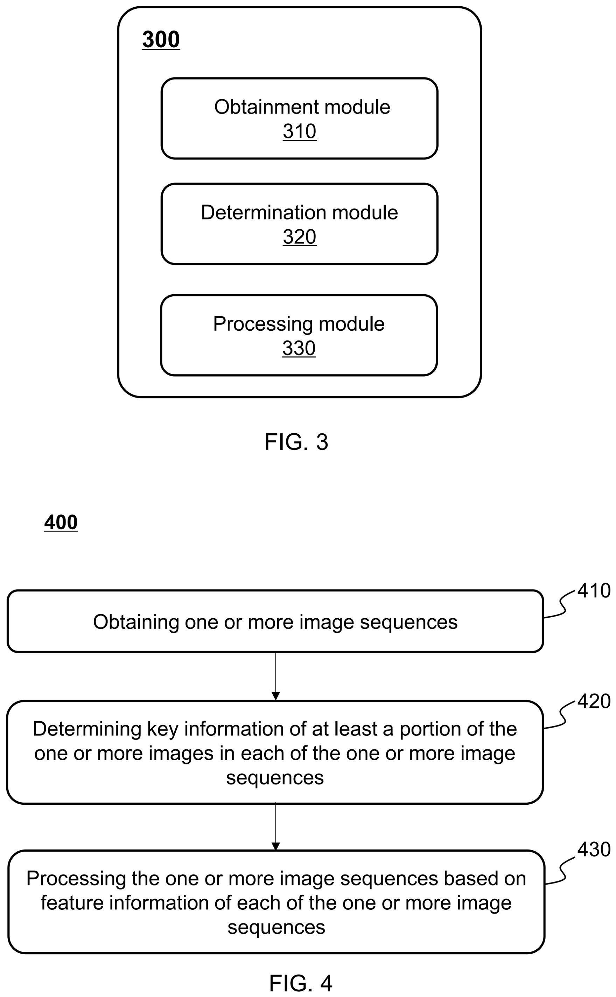

FIG. 3 is a block diagram illustrating a system for image processing according to some embodiments of the present disclosure. As shown in FIG. 3, a system for image processing 300 includes an obtainment module 310, a determination module 320, and a processing module 330. The obtainment module 310 is configured to acquire one or more image sequences. The determination module 320 is configured to determine key information of each image in one or more image sequences. The processing module 330 is configured to processing the one or more image sequences based on feature information of each of the one or more image sequences. More descriptions of the obtainment module 310, the determination module 320, and the processing module 330 may be found in FIG. 4 to FIG. 11 and related descriptions.

FIG. 4 is a flowchart illustrating an exemplary method for image processing according to some embodiments of the present disclosure. As shown in FIG. 4, a process 400 includes the following operations. In some embodiments, the process 400 is performed by the processing device 110 or the system for image processing 300.

In 410, one or more image sequences are obtained. In some embodiments, operation 410 is performed by the processing device 110 or the obtainment module 310.

The image sequence refers to an image set of at least one anatomical region of the imaging object acquired by performing a scan (e.g., a CT scan, an MRI scan, etc.) on the imaging object at a time point or a plurality of consecutive time points within a scan time period. Each of the one or more image sequences includes one or more images. For example, the image sequence includes a plurality of images of the same modality and the same anatomical region. As another example, the image sequence includes a plurality of images of the same modality and different anatomical regions.

The modality refers to a scanning manner used to acquire images. For example, the modality is the CT scan or the MRI scan, etc.

The image sequence is determined based on at least one three-dimensional (3D) image corresponding to at least one imaging object acquired by an imaging device (e.g., the imaging device 140).

The 3D image refers to a 3D image acquired by reconstructing (e.g., 3D reconstruction) scan data acquired by an imaging device (e.g., the imaging device 140) from an imaging object.

The scan data refers to raw data acquired when an imaging device performs a scan on an imaging object, for example, X-ray data acquired by a CT device, magnetic resonance signals acquired by an MRI device, etc. The scan data is 3D data. The scan data includes a plurality of voxels. Each voxel is data (e.g., X-ray data, the magnetic resonance signals, etc.) of tissue at a corresponding position of the voxel in the imaging object.

The 3D image includes a plurality of slice images. Each slice image is reconstructed based on sub-scan data corresponding to the slice image (the sub-scan data refers to a data block composed of part of the voxels of the scan data, for example, each sub-scan data includes 128×128×8 voxels of the scan data.) The sub-scan data is generated from scan data corresponding to tissue of the imaging object within a thickness range (e.g., 1 mm). The thickness refers to a thickness along an axial direction. The axial direction refers to a vertical axis of a human body. The thickness is a layer thickness of the slice image.

In some embodiments, the image sequence includes all slice images in a 3D image.

In some embodiments, the image sequence is acquired by sampling the 3D image of the imaging object. Based on this, the image sequence includes a portion of slice images acquired by sampling the 3D image.

The sampling refers to an operation of extracting a portion of slice images from different layers (i.e., different axial positions) of the 3D image based on a sampling interval. The sampling interval refers to a spacing between axial positions of the imaging object corresponding to adjacent slice images among the extracted slice images (or referred to as inter-slice spacing information). For example, taking a layer thickness of the slice image as 1 mm, if a sampling manner is to extract one slice image every four slice images (i.e., extract one slice image from every five adjacent slice images), then the sampling interval is 5 mm. The sampling interval may be preset. For example, the sampling interval is preset to 5 mm, 1 cm, or 2 cm, etc. It may be understood that, for the 3D image with a determined layer thickness, by using different sampling intervals, the axial positions and a count of the extracted slice images are different, thereby acquiring different image sequences.

An exemplary process for acquiring the image sequences is as follows. The processing device 110 or the obtainment module 310 first acquires an image sequence corresponding to the 3D image. The image sequence includes all slice images in the 3D image. Further, the processing device 110 or the obtainment module 310 samples the 3D image using a plurality of (taking 4 as an example) different sampling intervals, respectively, to acquire a plurality of (4) different image sequences. Based on this, the processing device 110 or the obtainment module 310 may acquire a total of 5 different image sequences.

In some embodiments, each image in each image sequence only includes a specific anatomical region or organ of the imaging object. In some embodiments, each image in each image sequence is a whole-body image of the imaging object.

Regarding a division of anatomical regions of the imaging object, exemplarily, a region above vertebra C1 (the first cervical vertebra) is defined as a head, a region between vertebra C1 and C7 (the seventh cervical vertebra) is defined as a neck, a region between a lower boundary of vertebra C7 and a lower boundary of a heart is defined as a chest, a region between the lower boundary of the heart and a lower boundary of the kidneys is defined as an abdomen, a region between the lower boundary of the kidneys and an end of a sacrum is defined as a pelvis, and a region below the end of the sacrum is defined a lower limbs. In some embodiments, the whole-body image includes images of at least three anatomical regions: the chest, the abdomen, and the pelvis. The imaging object may also be divided into the anatomical regions in other manners, which are not limited herein.

In some embodiments, the image sequences include a first image sequence and one or more second image sequences. The first image sequence includes a plurality of first images, and each of the one or more second image sequences includes a plurality of second images.

The processing device 110 or the obtainment module 310 may select one image sequence from the one or more image sequences as the first image sequence through various manners.

In some embodiments, the first image sequence is an image sequence with a largest AFOV among the one or more image sequences. Among the one or more image sequences, image sequences other than the first image sequence are second image sequences.

The AFOV may be represented by a spacing between axial positions of the imaging object corresponding to a first image and a last image in the image sequence. A larger spacing indicates a larger AFOV. For example, if a sequence includes 10 images, the AFOV of the sequence is a spacing between axial positions of the imaging object corresponding to the first image and the 10th image in the image sequence. As another example, if a sequence only includes one image, the AFOV of the sequence is 0.

The processing device 110 or the obtainment module 310 may calculate the AFOV of each image sequence, respectively, and determine an image sequence with the largest AFOV as the first image sequence. For example, the image sequence is an image sequence stored according to a digital imaging and communications in medicine (DICOM) standard. The processing device 110 or the obtainment module 310 extracts a inter-slice spacing information from a standard public tag defined in the DICOM standard of the image sequence. Subsequently, a formula AFOV = inter-slice spacing information×(N-1) is used to calculate a size of the AFOV of each image sequence, and the image sequence with the largest AFOV value is determined as the first image sequence. In the above formula, N denotes a count of slice images in each image sequence. The count of slice images refers to a count of images included in the image sequence. For example, if the first image sequence includes a total of 5 first images, then the count of slice images of the first image sequence is 5.

In some embodiments, the processing device 110 or the obtainment module 310 determines a sequence score of each of the one or more image sequences based on diversified features of the image sequence and a score determination model, and determines the first image sequence based on the sequence score of each of the one or more image sequences.

The diversified features reflect image data features of the image sequence. The diversified features include at least one of a inter-slice spacing information, a count of slice images, and a sequence quality score.

The sequence quality score reflects an image quality of the one or more images in the image sequence. The sequence quality score is determined based on a quality score of each image in the image sequence (i.e., the image quality score). As an example, the sequence quality score is a median or an average of quality scores of the images in the image sequence.

The image quality score reflects an image quality of a single image. A larger value of the image quality score indicates a better image quality of the corresponding image. As an example, a larger image quality score value indicates that the image has higher clarity, and better resolution, etc. Descriptions of a manner for determining the image quality score may be found in related descriptions later in the present disclosure.

In some embodiments, if an image sequence is a multi-temporal sequence (e.g., the image sequence is acquired based on perfusion CT or perfusion MRI), the diversified features further include a temporal feature of the image sequence. The multi-temporal sequence refers to an image sequence in which the temporal features of included images change sequentially and gradually. The temporal feature refers to a change in scan data (e.g., a tracer concentration, a geometric feature of an organ or a lesion tissue, etc.) of a corresponding region (e.g., a region of interest) in the images in the image sequence. As an example, the temporal feature is a change rate of the tracer concentration in a same region of interest in the images, or the change rate of a shape, a size, position coordinates, etc., of an organ or a lesion tissue in the images.

The score determination model is configured to process the diversified features of the image sequence to acquire the sequence score of the image sequence.

The score determination model is a machine learning model. As an example, the score determination model is a neural network model. In some embodiments, a machine learning algorithm used by the score determination model includes random forest or gradient boosting decision tree.

An input of the score determination model is the diversified features of the image sequence, and an output is the sequence score.

In some embodiments, before inputting the diversified features into the score determination model, the processing device 110 or the obtainment module 310 normalizes each feature in the diversified features (including the inter-slice spacing information, the count of slice images, the sequence quality score, and/or the temporal feature), and then uses the normalized diversified features as the input of the score determination model. Merely by way of example, the normalized diversified features is a diversified feature vector. Each dimension in the diversified feature vector corresponds to normalized inter-slice spacing information, count of slice images, sequence quality score, and/or temporal feature. Values of the aforementioned features are normalized to values within a preset value interval (e.g., [0, 1]). Specifically, the value of each feature is determined by mapping the inter-slice spacing information, the count of slice images, the sequence quality score, and the temporal feature into the preset value interval. Taking the inter-slice spacing information as an example, if the inter-slice spacing information is greater than or equal to a preset reference value for the inter-slice spacing information, the value of the inter-slice spacing information is mapped to 1. If the inter-slice spacing information is less than the preset reference value for the inter-slice spacing information, the value of the inter-slice spacing information is mapped to a ratio of the inter-slice spacing information to the reference value for the inter-slice spacing information. Taking the count of slice images as another example, if the count of slice images is greater than or equal to a preset reference count value, the value of the count of slice images is mapped to 1. If the count of slice images is less than the preset reference count value, the value of the count of slice images is mapped to a ratio of the count of slice images to the reference count value. A normalization manner for the sequence quality score and the temporal feature is similar to a normalization manner for the inter-slice spacing information or the count of slice images, which is not repeated here.

The score determination model may be acquired by training an initial machine learning model based on first sample data. The first sample data includes sample diversified features of a plurality of sample image sequences or sample diversified feature vectors of a plurality of sample image sequences. A label of the first sample data is a sample sequence score. The first sample data is input into the initial machine learning model. A value of a first loss function is determined based on an output of the initial score determination model and the label. The initial machine learning model is iteratively trained until a preset condition is satisfied (e.g., a value of the first loss function is less than a first loss threshold, or a count of iterations is greater than or equal to a first preset count, etc.), to acquire a trained score determination model. The first sample data may be determined based on the image sequences acquired from historical scanning processes. The label may be acquired based on manual annotation.

In some embodiments, after determining the sequence score of each image sequence, the processing device 110 or the obtainment module 310 determines the image sequence with a highest sequence score as the first image sequence.

In 420, key information of at least a portion of the one or more images in each of the one or more image sequences is determined. In some embodiments, operation 420 is performed by the processing device 110 or the determination module 320.

In some embodiments, the at least a portion of images in the one or more image sequences is acquired by sampling the a portion of images from each of the one or more image sequences. A manner of the sampling may be random sampling or uniform sampling, etc.

In some embodiments, the at least a portion of images in the one or more image sequences is all images in the one or more image sequences.

The key information of an image (e.g., a first image or a second image) reflects semantic features of the image.

In some embodiments, the key information at least including at least one of anatomical region information, disease information, image quality information, image modality information, an image sequence type, or contrast information. The anatomical region information refers to a name of a body part of an imaging object contained in an image. As an example, the anatomical region information is a neck, a chest, an abdomen, etc. The disease information of the image reflects a disease condition detected based on the image. As an example, the disease information includes disease name, whether a tumor exists, and whether an inflammation exists, etc. The image quality information reflects a quality of an image. As an example, the image quality information includes a quality score of the image, whether an artifact exists, whether distortion exists, etc. Descriptions of the quality score may be found in related descriptions earlier in the present disclosure. The image modality information reflects a scanning type used by an imaging device when scanning an imaging object to acquire the image. As an example, the modality information is a CT scan, or a MRI imaging, etc. The image sequence type refers to a type of scanning sequence used in an imaging (e.g., magnetic resonance imaging) process. As an example, The image sequence type is a Steady-State Free Precession (SSFP) sequence, a Diffusion Weighted Imaging (DWI), or a Short Tau Inversion Recovery (STIR) sequence, etc. The contrast information reflects whether a contrast intelligent agent is injected into an imaging object when scanning the imaging object to acquire the image.

In some embodiments, the key information of the image further includes organ information or phase information. The organ information refers to a name of an organ of an imaging object contained in an image. As an example, the key information is "lung, heart," "larynx," "kidney," etc. It should be noted that, as a same anatomical region includes more than one organ (e.g., the chest includes the heart, lungs, a liver, etc.), the organ information in the key information includes names of a plurality of organs. The phase information refers to a phase corresponding to an image when scanning the imaging object to acquire the image. As an example, if the imaging object is the heart, the phase may be a systolic phase or a diastolic phase.

In some embodiments, the processing device 110 or the determination module 320 determines the key information of each of the at least a portion of the one or more first images in the first image sequence, respectively.

In some embodiments, for each of the at least a portion of the one or more first images in the first image sequence, the processing device 110 or the determination module 320 obtain the key information of the first image by processing the first image through a key information extraction model.

The key information extraction model is a machine learning model. As an example, the key information extraction model is a deep learning model. Specifically, the key information extraction model may be a vision transformer (vit) model, a convolutional neural network (CNN) model, a deep neural network (DNN) model, etc., which is not limited herein. The key information extraction model is configured to process an image (e.g., the first image) to acquire the key information of the image. An input of the key information extraction model includes an image (e.g., the first image). An output of the key information extraction model includes the key information corresponding to the input image (e.g., the first image input).

In some embodiments, before inputting the first image into the key information extraction model, the processing device 110 or the determination module 320 performs pre-processing (referred to as first pre-processing hereinafter) on the first image. The first image after the first pre-processing is then input into the key information extraction model.

In some embodiments, the first pre-processing includes a size adjustment. as an example, the sizes of all the first images are adjusted to 224 pixels × 224 pixels.

In some embodiments, the first pre-processing includes a min-max normalization.

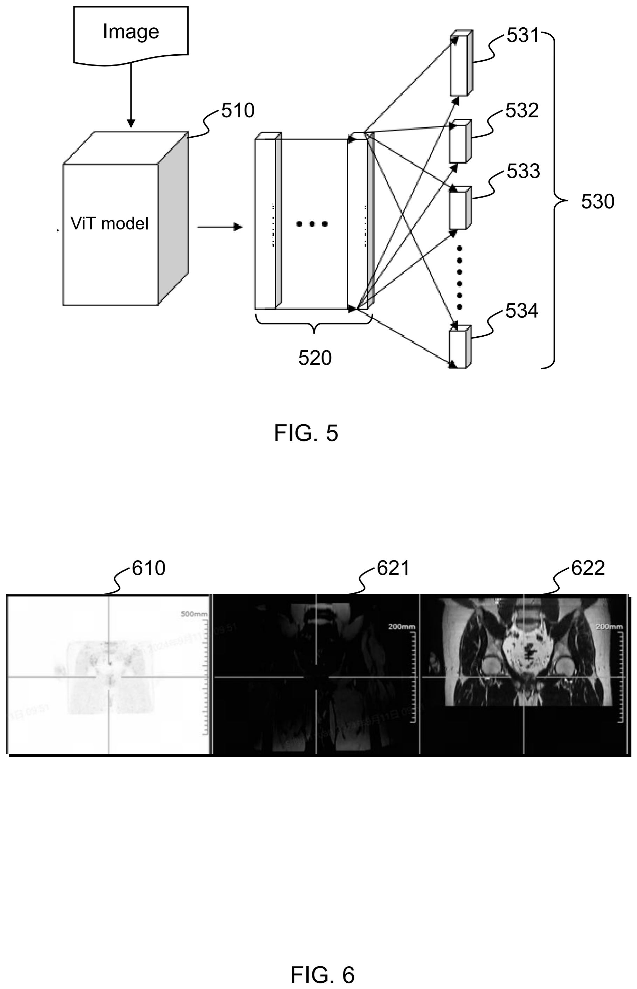

FIG. 5 is a model structure diagram illustrating a key information extraction model according to some embodiments of the present disclosure.

In some embodiments, as shown in FIG. 5, the key information extraction model includes a vision transformer (VIT) model 510, a feature processing layer 520, and a plurality of fully connected layer classifiers 530.

The VIT model 510 is configured to extract features of a first image. The VIT model 510 may capture global contextual relationships through a self-attention mechanism and output a high-dimensional feature vector rich in semantic information corresponding to the first image.

The feature processing layer 520 is configured to process the features (the high-dimensional feature vector) extracted by the vit model. The feature processing layer 520 may integrate and refine the high-dimensional feature vector. As an example, the feature processing layer 520 maps a feature dimension and converts the high-dimensional feature vector output by the VIT model 510 into a series of semantic feature vectors with clear semantic orientations, and the semantic feature vectors are suitable for classification tasks of the plurality of fully connected layer classifiers 530 downstream.

The semantic feature vectors may include an anatomical region vector and an organ vector. As an example, the anatomical region vector is (head: 0.02, neck: 0.01, chest: 0.14, abdomen: 0.81, pelvis: 0.02). Each value represents a probability that the first image contains the corresponding anatomical region. As another example, the organ vector is (lung: 0.10, heart: 0.05, liver: 0.95, stomach: 0.90). Each value represents a probability that the first image contains the corresponding organ.

In some embodiments, each fully connected layer classifier in the plurality of fully connected layer classifiers 530 is respectively configured to output one type of key information. As an example, as shown in FIG. 5, the plurality of fully connected layer classifiers 530 includes a fully connected layer classifier 531, a fully connected layer classifier 532, a fully connected layer classifier 533, a fully connected layer classifier 534, etc. The fully connected layer classifier 531 is configured to output anatomical region information. The fully connected layer classifier 532 is configured to output organ information. The fully connected layer classifier 533 is configured to output disease information. The fully connected layer classifier 534 is configured to output image quality information.

in some embodiments, each fully connected layer classifier in the plurality of fully connected layer classifiers 530 includes a classification head. As an example, the classification head is an anatomical region classification head, an organ classification head, or a disease information classification head, etc. The classification head is a specially designed module for outputting classification results (e.g., an anatomical region classification result, an organ classification result), and the classification head is placed at an end of a network structure of the key information extraction model. Each classification head is configured to receive the same semantic feature vector output by the feature processing layer 520. However, each classification head learns different threshold parameters. Each classification head is responsible for converting the semantic feature vector into a classification result within a corresponding category space through a nonlinear mapping and normalization process. The category space refers to a set composed of all possible classification results. Taking the anatomical region classification head as an example, the category space includes all possible body regions (e.g., the head, the neck, the chest, the abdomen, the pelvis, the lower limbs, etc.). The anatomical region classification result is at least one of the body regions in the category space. Taking the anatomical region classification result being the abdomen as an example, the anatomical region information determined by the fully connected layer classifier corresponding to the anatomical region classification head is the abdomen.

In some embodiments, each type of the key information is determined based on one or more fully connected layer classifiers. As an example, as shown in fig. 5, the classification head of the fully connected layer classifier 531 is an anatomical region classification head configured to determine an anatomical region classification result (i.e., the anatomical region information). The classification heads of the fully connected layer classifiers 532, 533, and 534 are all organ classification heads. The classification head of the fully connected layer classifier 532 is an organ classification head corresponding to "lung" and is configured to determine whether the organ classification result (i.e., the organ information) includes the lung. The classification head of the fully connected layer classifier 533 is an organ classification head corresponding to "heart" and is configured to determine whether the organ classification result includes the heart. The classification head of the fully connected layer classifier 534 is an organ classification head corresponding to "kidney" and is configured to determine whether the organ classification result includes the kidney.

Merely by way of example, assuming that the threshold parameter of the anatomical region classification head is 0.7 and the threshold parameters of the respective organ classification heads are all 0.8, and still taking the region vector as (head: 0.02, neck: 0.01, chest: 0.14, abdomen: 0.81, pelvis: 0.02), the organ vector as (lung: 0.10, heart: 0.05, liver: 0.95, stomach: 0.90) as an example, in the anatomical region vector, only the probability of the abdomen (0.81) is greater than the threshold parameter of the anatomical region classification head (0.7). Therefore, the anatomical region information output by the anatomical region classification head is the abdomen. In the organ vector, the probability of the liver (0.95) and the probability of the stomach (0.9) are greater than the threshold parameter of the organ classification head (0.8). Therefore, the organ information output by the plurality of fully connected layer classifiers 530 includes the liver and the stomach.

The key information extraction model may be acquired by training an initial key information extraction model based on second sample data. The second sample data includes a plurality of first sample images. A label of the second sample data includes sample key information of each first sample image. The second sample data is input into the initial key information extraction model. A value of a second loss function is determined based on an output of the initial key information extraction model and the label. The initial key information extraction model is iteratively trained until a preset condition is satisfied (e.g., the value of the second loss function is less than a second loss threshold, or the count of iterations is greater than or equal to a second preset count, etc.) Then a trained key information extraction model is acquired. The second sample data may be determined based on images from a historical image sequence acquired from a historical scanning process. The label may be acquired based on manual annotation.

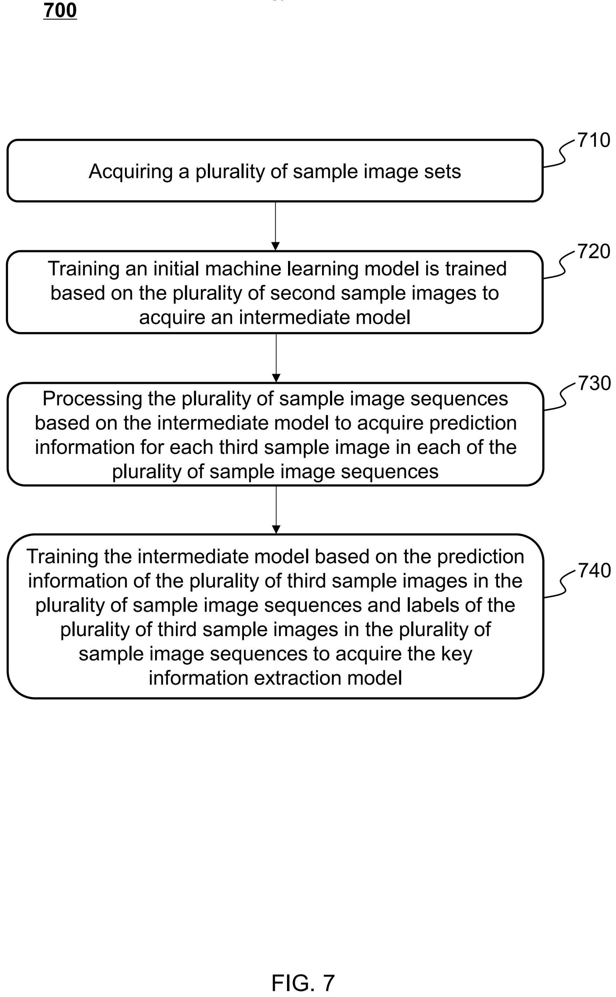

In some embodiments, a training process of the key information extraction model includes: acquiring a plurality of sample image sets, the plurality of sample image sets including a plurality of second sample images and a plurality of sample image sequences, each sample image sequence of the plurality of sample image sequences including a plurality of third sample images; training an initial key information extraction model based on the plurality of second sample images to acquire an intermediate model; processing the plurality of sample image sequences based on the intermediate model to acquire predicted information of each third sample image in each sample image sequence; and training the intermediate model based on the predicted information and labels of the plurality of third sample images in the plurality of sample image sequences to acquire the key information extraction model. More descriptions of this embodiment may be found in FIG. 7 and related descriptions.

In some embodiments of the present disclosure, the key information extraction model, which includes the ViT model, the feature processing layer, and a plurality of fully connected layer classifiers, is configured to efficiently extract a global feature and simultaneously output a plurality of types of the key information, thereby improving a recognition accuracy and task parallelism capability of the model.

In some embodiments, for each of the at least a portion of the one or more first images in the first image sequence, the processing device 110 or the determination module 320 determines an image quality score of the first image via a quality evaluation model. Descriptions of the image quality score may be found in related descriptions above.

The quality evaluation model is a machine learning model, e.g., a neural network model, etc. The quality evaluation model is configured to process an image (e.g., a first image) to acquire the image quality score of the image. An input of the quality evaluation model includes an image (e.g., a first image), and an output of the quality evaluation model includes the image quality score of the input first image.

The quality evaluation model may be acquired by training an initial machine learning model based on third sample data. The third sample data includes a plurality of fourth sample images, and a label of the third sample data include a sample quality score of each fourth sample image. The third sample data is input into the initial machine learning model. A value of a third loss function is determined based on an output of the initial machine learning model and the label. The initial machine learning model is iteratively trained until a preset condition is satisfied (e.g., a value of the third loss function is less than a third loss threshold, or an iteration count is greater than or equal to a third preset count, etc.) to acquire a trained quality evaluation model. The third sample data may be determined based on images in the historical image sequences acquired from the historical scan processes, and the label may be acquired based on manual annotation.

In some embodiments, for each of the at least a portion of the one or more first images in the first image sequence, after determining the quality score of the first image, the processing device 110 or the determination module 320 select a processing path for the first image based on the quality score to determine the key information of the first image. Different processing paths correspond to different ways of determining the key information of the first image.

In response to the quality score being greater than or equal to a first threshold, the processing device 110 or the determination module 320 processes the first image through the key information extraction model to acquire the key information of the first image. Descriptions of the key information extraction model may be found in related descriptions above.

In response to the quality score being less than the first threshold and greater than a second threshold, the processing device 110 or the determination module 320 performs image enhancement processing on the first image to acquire an enhanced first image. Then, the enhanced first image is processed through the key information extraction model to acquire the key information of the first image. The image enhancement processing includes a histogram equalization, a Gamma correction, a sharpening, a processing using a Retinex algorithm, a guided filtering processing, etc., which are not limited herein.

In response to the quality score being less than or equal to the second threshold, the processing device 110 or the determination module 320 selects a substitute image corresponding to the first image from the first image sequence. Then, the substitute image is processed through the key information extraction model to acquire the key information of the first image.

In some embodiments, the substitute image is any image in an adjacent image group. The adjacent image group includes at least one other first image (hereinafter referred to as a reference first image) in the first image sequence. An image interval between the reference first image and the first image for which the key information needs to be determined (hereinafter referred to as a first image to be processed) is less than or equal to an interval threshold. The image interval refers to a count of first images between the reference first image and the first image to be processed. The interval threshold may be preset, e.g., the interval threshold is preset as 1, 2, 3, etc.

In some embodiments, the processing device 110 or the determination module 320 determines the quality score of each reference first image within the adjacent image group through the quality evaluation model. If the quality scores of the reference first images are all less than or equal to the second threshold, further processing (e.g., an interpolation, an image fusion, etc.) is performed on at least one reference first image in the adjacent image group to generate an estimated image, and the estimated image is used as the substitute image.

The first threshold is greater than the second threshold.

In some embodiments, values of the first threshold and the second threshold are both preset.

In some embodiments, the first threshold and the second threshold are determined based on first user feedback data.

The first user feedback data refers to quality evaluations of the first image or the key information of the first image by a user (e.g., a physician or a researcher) when viewing the first image or the key information of the first image. The first user feedback data may include a user comment. Merely by way of example, the user comment is "good image quality," "poor image quality," "severe image distortion," etc.

Merely by way of example, in response to determining that the user comment for the first image with a high quality score (the quality score is greater than the first threshold) frequently includes "poor image quality," the processing device 110 or the determination module 320 increases the first threshold, so that more first images undergo the image enhancement processing before being input to the key information extraction model. Conversely, in response to a determining that user comment for the first image with a high quality score rarely includes "poor image quality," the processing device 110 or the determination module 320 decreases the first threshold to reduce a redundant processing and lower a computational cost. As another example, in response to determining that the user comment for the first image frequently includes "severe image distortion," the processing device 110 or the determination module 320 increases the second threshold, so that more medium-to-low quality images (the quality scores less than the first threshold and greater than the first threshold) are replaced with the substitute images.

In some embodiments of the present disclosure, by introducing the quality evaluation for the first image and selecting different processing paths based on the quality score, an accuracy and robustness of key information extraction are improved, thereby avoiding a misjudgment of the key information caused by inputting low-quality images into the key information extraction model.

In some embodiments, for each of at least a portion of the one or more second images in each of the one or more second image sequences respectively, the processing device 110 or the determination module 320 determines a matching result between the second image and each of the at least a portion of the one or more first images in the first image sequence, and determines the key information of the second image based on the matching result and the key information of each of the at least a portion of the one or more first images in the first image sequence.

In some embodiments, the processing device 110 or the determination module 320 determines a matching result between a second position of the second image and a first position of each of the at least a portion of the one or more first images in the first image sequence.

The first position is a coordinate position in a spatial coordinate system of a first imaging portion of the imaging object represented in the first image. The second position is a coordinate position in the spatial coordinate system of a second imaging portion of the imaging object represented in the second image. A coordinate origin of the spatial coordinate system is located within the imaging object.

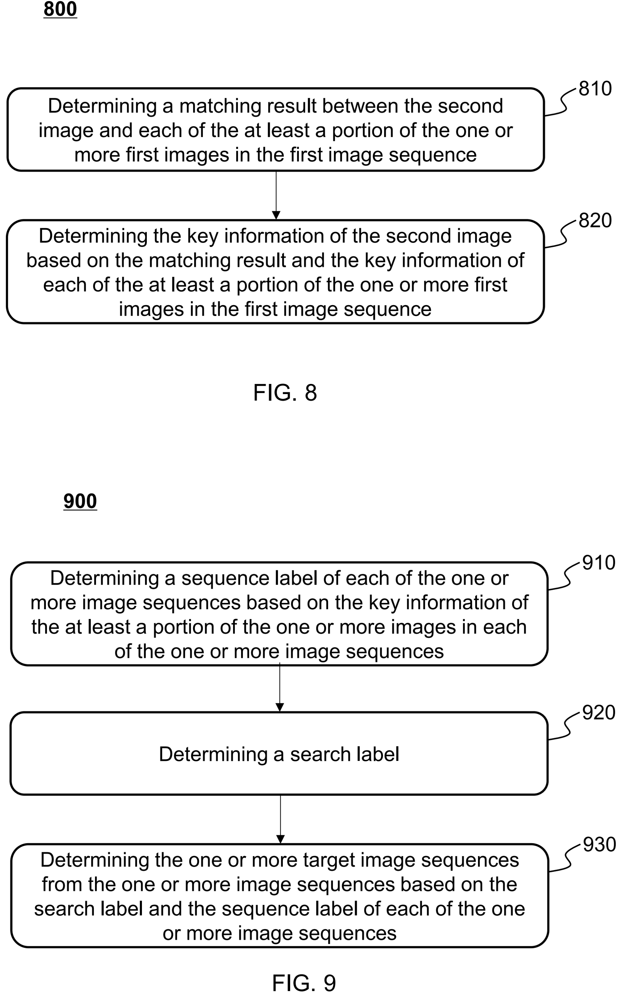

More descriptions regarding the above embodiments may be found in FIG. 8 and the related descriptions.

In 430, the one or more image sequences are processed based on feature information of each of the one or more image sequences. In some embodiments, operation 430 is performed by the processing device 110 or the processing module 330.

The feature information of each of the one or more image sequences reflects a feature of the image sequence itself or features of the at least a portion of the one or more images in the image sequence. The feature information of each of the one or more image sequences includes the key information of the at least a portion of the one or more images in the image sequence.

In some embodiments, the processing device 110 or the processing module 330 determines one or more target image sequences from the one or more image sequences based on the key information of the at least a portion of the one or more images in each of the one or more image sequences.

In some embodiments, the feature information of each of the one or more image sequences further includes a sequence label the image sequence
In some embodiments, the processing device 110 or the processing module 330 determines a sequence label of each of the one or more image sequences based on the key information of the each image in the one or more image sequences. The processing device 110 or the processing module 330 determines a search label. The processing device 110 or the processing module 330 determines the one or more target image sequences from the one or more image sequences based on the search label and the sequence label of each of the one or more image sequences. More descriptions regarding the above embodiments may be found in FIG. 9 and the related descriptions.

In some embodiments, the one or more target image sequences include a first target image sequence and at least one second target image sequence.

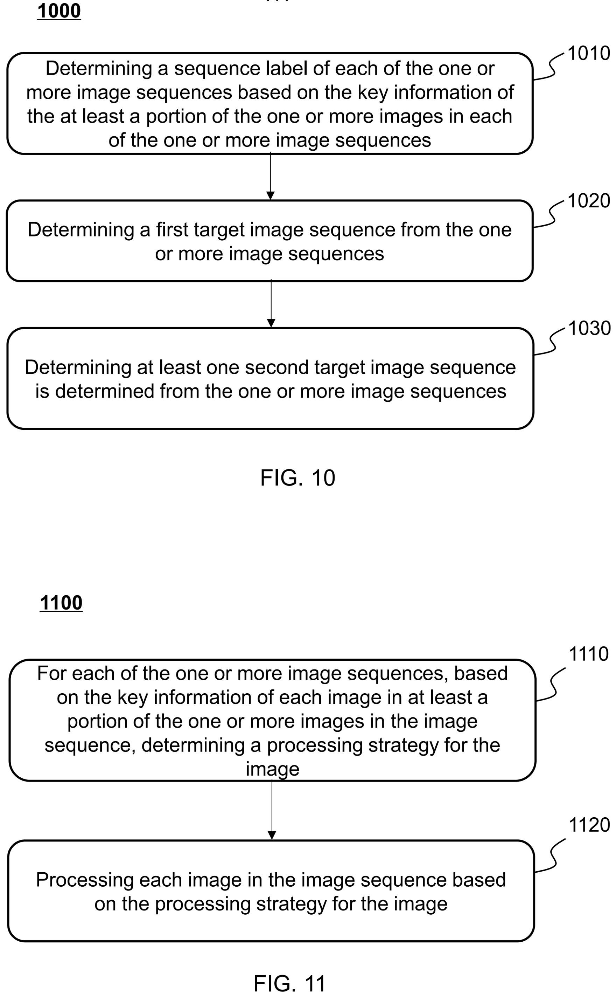

In some embodiments, the processing device 110 or the processing module 330 determines a sequence label of each of the one or more image sequences based on the key information of the each image in the one or more image sequences. The processing device 110 or the processing module 330 determines the first target image sequence from the image sequences. The processing device 110 or the processing module 330 determines a search label corresponding to the first target image sequence. The processing device 110 or the processing module 330 determines at least one second target image sequence from the one or more image sequences based on the search label and the sequence label of each of the one or more image sequences. More descriptions regarding the above embodiments may be found in FIG. 10 and the related descriptions.

In some embodiments, the processing device 110 or the processing module 330 sends the one or more target image sequences to a user terminal for display.

In some embodiments, a display device (e.g., a display screen) of the user terminal displays the one or more target image sequences in a side-by-side layout. A specific layout is shown in FIG. 6. FIG. 6 is a schematic diagram illustrating a display layout of one or more target image sequences according to some embodiments of the present disclosure. FIG. 6 shows a first target image sequence 610 and two second target image sequences (a second target image sequence 621 and a second target image sequence 622). As shown in FIG. 6, the first target image sequence 610, the second target image sequence 621, and the second target image sequence 622 are displayed in a horizontal side-by-side layout. The first target image sequence 610 is located on a far left, the second target image sequence 622 is located on a far right, and the second target image sequence 621 is located between the first target image sequence 610 and the second target image sequence 622.

Optionally, layout rules for the one or more target image sequences may be flexibly changed to adapt to diversified clinical application scenarios.

As another example, a user rotates the first target image sequence and the at least one second target image sequence to acquire any image in the first target image sequence or the at least one second target image sequence. Alternatively, the first target image sequence and the at least one second target image sequence are displayed in a form of slice images. The user views each image on a display interface of the display device through a page-turning operation.

In some embodiments of the present disclosure, the above manner determines the key information of images in the one or more image sequence. Compared to manual determination, this manner can significantly reduce a probability of errors when acquiring the at least one second target image sequence based on the key information, thereby greatly improving the accuracy and relevance of the image sequences displayed for the user. Furthermore, by displaying the first target image sequence and the at least one second target image sequence associated with the first target image sequence, an operation of separately processing each image sequence is saved, thereby reducing an operational complexity of the image display process.

In some embodiments, the processing device 110 or the processing module 330 determines at least one target application scenario adapted to the one or more image sequences based on the key information of at least a portion of images in the one or more image sequences.

In some embodiments, for each image sequence in the one or more image sequences, the processing device 110 or the processing module 330, based on the key information of each of the at least a portion of images in the image sequence, performs pre-processing on the image sequence to obtain a pre-processing result corresponding to each image in the at least a portion of the one or more images. Subsequently, the processing device 110 or the processing module 330 may update the key information of each of the at least a portion of images in the image sequence based on the pre-processing result.

The pre-processing (referred to as second pre-processing hereinafter) includes at least one operation of image segmentation, image alignment, image classification, or artifact removal.

In some embodiments, the processing device 110 or the processing module 330 determines a type of a pre-processing (second pre-processing) operation for each of the at least a portion of images in each of the one or more image sequences based on a correspondence table. The correspondence table records key information, whether a pre-processing operation is required for each piece of key information, and a type of a pre-processing required for each piece of key information. A content of the correspondence table may be preset.

Merely by way of example, for an image whose modality information is "CT scan," the processing device 110 or the processing module 330 performs a multi-organ image segmentation on the image. As a further example, taking an abdominal CT image as an example, if the modality information of the image is "CT scan," a full-image organ and tumor segmentation is performed on the image to acquire the pre-processing result (referred to as second pre-processing result hereinafter) of the image (also referred to as pre-processing image). As the abdominal CT enhanced scans are mostly performed to examine abdominal tumors, performing the image segmentation facilitates tumor viewing, localization, measurement, etc., thereby providing a foundation for subsequent more precise tumor analysis.

As another example, for an image whose image quality information is "presence of artifacts," the processing device 110 or the processing module 330 performs an artifact removal operation on the image to acquire the pre-processing result (i.e., second pre-processing result) of the image (also referred to as pre-processing image).

In some embodiments, if the the one or more image sequences are multi-time sequence (e.g., a myocardial perfusion image sequence), the processing device 110 or the processing module 330 also performs alignment on each image in the one or more image sequences. For example, each image is aligned with its preceding image arranged in the image sequence to acquire a displacement of each image relative to its preceding image arranged in the image sequence.

Correspondingly, the second pre-processing result includes an image segmentation result (e.g., segmentation masks), an image alignment result (e.g., the image sequence spatially aligned based on the aforementioned displacement), a classification result, or an artifact removal result (e.g., an image with reduced artifacts or without artifacts).

In some embodiments, for each of the at least a portion of the one or more images in an image sequence, the processing device 110 or the processing module 330 processes the image and the second pre-processing result corresponding to the image to obtain updated key information of the image. For example, the processing device 110 or the processing module 330 processes an image and the pre-processing result using the key information extraction model to acquire updated key information of the image.

In some embodiments, for the second pre-processing result of each of the at least a portion of the one or more images in an image sequence, the processing device 110 or the processing module 330 encodes a pre-processed result to acquire an encoded feature. In some embodiments, the processing device 110 or the processing module 330 processes the encoded feature (e.g., the encoded feature of the segmentation mask) and an original image (e.g., processing the original image and the segmentation mask of the original image) to acquire updated key information of the image. In some embodiments, the processing device 110 or the processing module 330 processes the encoded feature using the key information extraction model (e.g., the encoded feature of the image after artifact removal) to acquire the updated key information of the image.

The aforementioned encoding process includes a feature extraction and a feature transformation. In some embodiments, the aforementioned encoding process further includes a modality encoding.

The feature extraction includes: processing the second pre-processing result using a feature extraction model to acquire a feature vector corresponding to the second pre-processing result. The feature extraction model is a trained machine learning model, e.g., a sub-architecture model based on a CNN or a ViT. An input of the feature extraction model includes the second pre-processing result, and an output includes the feature vector. For example, for the segmentation mask (e.g., a binary image marking an organ region), the feature extraction model extracts a shape, a texture, and/or spatial features from the segmentation mask (e.g., a regularity of a tumor boundary, a relative position of the organs) and output the feature vector. The feature vector refers to a vector reflecting a pre-processing result (e.g., a shape feature, a texture feature, or a spatial feature of the segmentation mask). A value of each dimension of the feature vector is a numeric representation of the feature of the pre-processing result (e.g., a shape encode, a texture encode, etc.).

The feature transformation includes: standardizing and normalizing the feature vector. The feature transformation may be performed based on a dimensionality reduction technique (e.g., PCA) or a regularization technique.

The modality encoding includes: adding a modality identifier (e.g., a position encoding or a label encoding) to the second pre-processing result to clearly indicate a source of a feature (e.g., distinguishing whether the feature comes from the segmentation mask or the original image), thereby avoiding a feature confusion.

In some embodiments of the present disclosure, the pre-processing is performed when determining the key information, and the key information is updated based on the pre-processing result, thereby improving an accuracy of the key information and a reliability of subsequent application scenario matching results.

In some embodiments, the processing device 110 or the processing module 330 uses a scenario determination model to process the key information and the auxiliary information of each of the at least a portion of images in each of the one or more image sequences to acquire at least one target application scenario.

The scenario determination model is a trained machine learning model (e.g., a neural network model, etc.). An input of the scenario determination model includes the key information and the auxiliary information of the at least a portion of images. An output of the scenario determination model includes at least one target application scenario adapted to the one or more image sequences.

In some embodiments, the feature information of each of the one or more image sequences further includes auxiliary information of the at least a portion of the one or more images in each of the one or more image sequences.

In some embodiments, the processing device 110 or the processing module 330 selects at least one candidate application scenario adapted to the one or more image sequences from a plurality of candidate application scenarios as the at least one target application scenario based on the key information and auxiliary information of the at least a portion of images in each of the one or more image sequences.

The auxiliary information refers to information other than the key information that is related to one or more imaging objects or one or more imaging processes of the one or more image sequences.

The auxiliary information includes metadata of the at least a portion of the one or more images in each of the one or more image sequences and/or clinical data information of an imaging object corresponding to each of the one or more image sequences.

The metadata of an image reflects a specification or one or more parameter of the image in the one or more image sequences. The metadata of an image includes a modality, an image orientation, a pixel spacing, a slice thickness, a inter-slice spacing information, an acquisition date, manufacturer information of an imaging device for acquiring the image, etc., of the image.

The image orientation reflects a position feature of the image in a 3D space (e.g., the spatial coordinate system described above). The position feature includes an image position and an image orientation, etc. The image position refers to position coordinates of a reference point (a geometric center point) of an imaging part of the imaging object corresponding to the image in the spatial coordinate system. The image orientation may be represented through angles from a row vector and a column vector to a coordinate axis (e.g., Z-axis) of the spatial coordinate system.

The pixel spacing of an image refers to a spacing between two portions of an imaging object represented by two adjacent pixels in the image.

The manufacturer information of an image refers to information related to a manufacturer of an imaging device, e.g., a name or a code of the manufacturer, that is used to acquire the image.

In the metadata, the modality, the image orientation, the pixel spacing, the slice thickness, and the inter-slice spacing information reflect a clarity of an imaging site of the imaging object within the image. The acquisition date reflects a timeliness of the image. The manufacturer information reflects format information of the image.

In some embodiments, the one or more images in the one or more image sequences are stored according to a DICOM standard. The processing device 110 or the processing module 330 extracts metadata tags of the one or more images in the one or more image sequences. The processing device 110 or the processing module 330 reads the metadata tag of each image to acquire tag information of each image in the one or more image sequences.

In some embodiments, if DICOM information of the image is lost, or if the images in the image sequence are not stored according to the DICOM standard, the processing device 110 or the processing module 330 uses the key information extraction model described above to determine the metadata of the image. In this case, an input of the key information extraction model includes the image (e.g., the image in the one or more image sequences). An output of the key information extraction model includes the metadata of the image.

The clinical data information refers to personalized information related to the imaging object itself. Merely by way of example, the clinical data information includes basic information of the imaging object (e.g., a gender, an age, a height, a weight, etc., of the imaging object), a description of a scan site (e.g., whether a lesion visible to naked eyes exists at the scan site), an examination purpose (e.g., a disease type to be determined based on the image sequence containing the image), treatment information (e.g., a currently taken drug, a treatment project that has been received, etc.), etc.

The application scenario refers to a computer platform or an application program capable of analyzing or processing an image sequence. As another example, the application scenario is a lung nodule detection program, a chest volume measurement program, a coronary artery analysis program, etc.

The target application scenario refers to an application scenario most suitable for processing images in the one or more image sequences.

In some embodiments, the processing device 110 or the processing module 330 selects, via an intelligent agent and based on the key information and the auxiliary information of the at least a portion of images in the one or more image sequences, selects at least one candidate application scenario adapted to the one or more image sequences from a plurality of candidate application scenarios as the at least one target application scenario.

The intelligent agent refers to a software system constructed based on an artificial intelligence model and having an autonomous decision-making and task-processing mechanism. The intelligent agent is capable of understanding semantics of input information (e.g., the key information and the auxiliary information of each image in the image sequence). The intelligent agent performs a comprehensive, multi-dimensional analysis on the input information (e.g., based on preset inference rules or a large language model (LLM)). The intelligent agent then autonomously determines an application scenario (i.e., the target application scenario) or a processing algorithm that best matches the one or more image sequences from the plurality of candidate application scenarios.

In some embodiments, the intelligent agent processes the key information and the auxiliary information of the at least a portion of images in the one or more image sequences and the plurality of candidate application scenarios based on preset inference rules or the LLM to acquire an adaptation degree of each candidate application scenario with the one or more image sequences. The adaptation degree reflects a degree of adaptation between the candidate application scenario and the one or more image sequences. A higher value of the adaptation degree indicates a higher degree of adaptation between the candidate application scenario and the one or more image sequences.

In some embodiments, the intelligent agent determines m count of candidate application scenarios with the greatest values of the adaptation degree as the at least one candidate application scenario adapted to the one or more image sequences, m is a positive integer, and m is greater than or equal to 1 and less than a count of the candidate application scenarios.

In some embodiments, if the key information or the auxiliary information of some images in the at least a portion of images is missing, the intelligent agent completes the key information or the auxiliary information of the images with missing information based on context (e.g., images adjacent to the images with missing information). Alternatively, the intelligent agent sends an information completion request to a user terminal to complete the key information or the auxiliary information of the images with missing information based on the key information or the auxiliary information input by the user.

In some embodiments, as the intelligent agent operates, the intelligent agent adjusts the inference rules or the LLM based on historical operation records (recording historical image sequences that have been processed, and determined historical target application scenarios) and user usage feedback. For example, according to the above historical operation records, after processing the historical image sequences containing the image whose key information is "chest region-enhanced CT," the intelligent agent outputs result (the at least one historical target application scenario determined by the intelligent agent that is adaptable with the historical image sequences) that mostly includes coronary artery analysis programs (e.g., among the output results acquired from processing 10 historical image sequences, 9 historical image sequences contain coronary artery analysis programs). Then, when the intelligent agent subsequently processes the image sequences containing the key information "chest region-enhanced CT", the intelligent agent increases an output priority of the coronary artery analysis program, thereby increasing a probability that at least one of the determined target application scenarios includes the coronary artery analysis program.

In some embodiments, the intelligent agent determines whether the key information of each image in the at least a portion of images in each of the one or more image sequences satisfies a processing condition.

The processing condition refers to a condition for determining whether the image can be processed by the intelligent agent. In some embodiments, the processing condition is that the key information is not missing and/or the key information is correct. Whether the key information of an image is correct may be determined based on the key information of images in an adjacent image group of the image. For example, if the anatomical region information of the image is the head region, and the anatomical region information of the images in the adjacent image group of the image is the chest region, then the key information of the image is considered incorrect. The adjacent image group includes at least one other image in the image sequence whose image interval from the image being determined for whether it satisfies the processing condition is less than or equal to an interval threshold.

In some embodiments, in response to determining that the key information of an image does not satisfy the processing condition, the intelligent agent triggers pre-processing or information supplementation to update the key information of the image. Descriptions of the pre-processing may be found in the related descriptions above.

The information supplementation refers to a process of completing or correcting the key information of the image in the one or more image sequences that does not satisfy the processing condition (hereinafter referred to as an image to be supplemented).

In some embodiments, the intelligent agent performs the information supplementation on the key information of the image to be supplemented based on other image sequences (hereinafter referred to as first matching image sequences) having the highest matching degree with the image sequence containing the image to be supplemented (hereinafter referred to as an image sequence to be supplemented).

Merely by way of example, the intelligent agent first performs alignment on the image sequence to be supplemented and the first matching image sequence to acquire a spatial position transformation matrix. After performing the alignment, the intelligent agent matches (e.g., an intensity normalization or a histogram matching) the image to be supplemented with each image in the first matching image sequence to acquire a matching result. Then, the intelligent agent may use the key information of the image among the plurality of images in the first matching image sequence whose matching result is consistent as the key information of the image to be supplemented. The alignment process includes: establishing a global alignment between the image to be supplemented and each image in the first matching image sequence using fast rigid/affine alignment, and then applying refined non-rigid alignment for local structures to eliminate differences in organ displacement or respiratory motion. More descriptions of the matching result and the spatial position transformation matrix may be found in FIG. 8 and the related descriptions. More descriptions of the matching degree may be found in related descriptions in FIG. 10 and the related descriptions.

In some embodiments, the intelligent agent reselects at least one candidate application scenario adapted to the one or more image sequences based on the updated key information.

In some embodiments of the present disclosure, through a self-assessment mechanism, the intelligent agent automatically triggers the information supplementation or the pre-processing when determining that the key information does not satisfy the processing condition. This enables a dynamic optimization of its own decision-making process, thereby achieving an autonomous re-evaluation and correction of the key information, and improving the accuracy of the subsequently selected at least one candidate application scenario.

In some embodiments of the present disclosure, using the intelligent agent for multi-scenario matching decisions (selecting the at least one candidate application scenario adapted to the one or more image sequences from the plurality of candidate application scenarios) enables the system (e.g., the system for application scenario matching 1000) to autonomously select the most appropriate target scenario from the plurality of candidate applications based on semantic understanding and reasoning capabilities. This improves the efficiency of determining the target application scenario and the adaptation degree between the determined target application scenario and the one or more image sequences.

The candidate application scenario refers to an application scenario that the user terminal is able to support. The plurality of candidate application scenarios may be preset in a scenario database. The scenario database may be configured in a storage device (e.g., the storage device 130).

In some embodiments, the processing device 110 or the processing module 330 matches the key information and the auxiliary information of images in a set of images with constraint conditions of the plurality of candidate application scenarios, to determine at least one candidate application scenario adapted to the one or more image sequences from the plurality of candidate application scenarios as at least one target application scenario. The set of images includes the at least a portion of images in each of the one or more image sequences.

The constraint condition refers to a screening condition used to determine whether the candidate application scenario is adapted to the one or more image sequences. The constraint condition may be preset. For example, for each candidate application scenario, the user sets a corresponding constraint condition based on a clinical requirement and a test performance. The constraint condition of a candidate application scenario may represent a constraint specification for images used by the candidate application scenario.

In some embodiments, the constraint condition of each of the plurality of candidate application scenarios includes at least one of the following items (C11 to C15):
C11: The set of images includes images including a target object. The target object is an application object of the candidate application scenario (e.g., an imaging anatomical region or an organ of the imaging object). Taking the candidate application scenario of an MR breast evaluation application as an example, the application object of the MR breast evaluation application is a breast, then the target object is the breast. Based on this, if the set of images includes one or more images containing the breast (i.e., images whose organ information is the breast), then the one or more image sequences satisfy constraint condition C11.
C12: A proportion of images including a target object in the set of images is greater than or equal to a first proportion threshold. Taking the candidate application scenario of a CT brain perfusion application as an example, the application object of the CT brain perfusion application is the head, so the target object is the head. Based on this, if, a proportion of images containing the head (i.e., images whose anatomical region information is the head region) among images in the set of images is greater than or equal to the first proportion threshold, then the one or more image sequences satisfy constraint condition C12. The first proportion threshold may be preset, e.g., the first proportion threshold is 50%, 70%, or 80%, etc.
C13: A target object is an object with a largest proportion in all objects contained in the set of images. Taking the candidate application scenario of a CT heart application as an example, the application object of the CT heart application is the heart, so the target object is the heart. Based on this, if, among all objects included in the set of images, the heart is the object with the greatest proportion, then the one or more image sequences satisfy constraint condition C13.

A proportion of each object included in the set of images is determined based on a frequency of occurrence of the object in the set of images, and a total frequency of occurrence (a sum of frequencies of occurrence) of all objects in the set of images. The frequency of occurrence of an object is counted in the following manner: an initial frequency of occurrence of the object is set to 0. For each image in the set of images that includes the object, the frequency of occurrence of the object is increased by 1. Merely by way of example, assuming that the set of images includes a total of 3 images (denoted as image X, image Y, and image Z, respectively), and image X includes objects of the heart, a kidney, and a lung (a total of 3 objects), image Y includes objects of the heart and the kidney (a total of 2 objects), and image Z includes objects of the heart, the lung, and a liver (a total of 2 objects), then a count of occurrences of the heart is 3, counts of occurrences of the kidney and the lung are 2, and a count of occurrence of the liver is 1. A total frequency of occurrence of all objects is 3+2+2+1=8. Then, a proportion of the heart is 3/(3+2+2)=3/8, a proportion of the kidney or the lung is 2/(3+2+2)=2/8, and a proportion of the liver is 1/(3+2+2)=1/8.

C14: An actual physical size of a target object in at least one image in the images including the target object is greater than or equal to a size threshold. The size threshold may be preset, e.g., the size threshold is preset to 8 cm, 10 cm, or 12 cm, etc.

C15: A proportion of images having a modality being a preset modality in the set of images is greater than a second proportion threshold. The preset modality is a modality required by the candidate application scenario. Taking the candidate application scenario being an MR breast evaluation application as an example, a modality required by the MR breast evaluation application is a CT plain scan or a CT enhanced scan. If among the set of images, a proportion of images having a modality being the CT plain scan or the CT enhanced scan is greater than the second proportion threshold, then the one or more image sequences satisfy constraint condition C15.

In some embodiments, the constraint condition for each of the plurality of candidate application scenarios further includes other constraint conditions (e.g., a count of slice images in each of the one or more image sequences is greater than a quantity threshold), which is not limited herein.

The above constraint conditions can ensure that, when the images in the one or more image sequences are processed by a selected target application scenario, the size of the target object satisfies the requirements, or the count of images is sufficient, or the modality can satisfies requirements of the target application scenario, thereby ensuring accuracy and adaptability when applying the images in the one or more image sequences to the target application scenario.

In some embodiments, if the one or more image sequences satisfy any one of at least one constraint condition of the candidate application scenario, the processing device 110 or the processing module 330 determines that the candidate application scenario is compatible with the one or more image sequences, and the candidate application scenario serves as the target application scenario. Taking the candidate application scenario whose constraint conditions include C11, C13, and C14 as an example, if the one or more image sequences satisfy any one of C11, C13, and C14, the processing device 110 or the processing module 330 determines that the candidate application scenario is compatible with the one or more image sequences.

In some embodiments, if the one or more image sequences satisfy all constraint conditions of the candidate application scenario, the processing device 110 or the processing module 330 determines that the candidate application scenario is compatible with the one or more image sequences, and the candidate application scenario serves as the target application scenario. Still taking the candidate application scenario whose constraint conditions include C11, C13, and C14 as an example, if the one or more image sequences simultaneously satisfy C11, C13, and C14, the processing device 110 or the processing module 330 determines that the candidate application scenario is compatible with the one or more image sequences; if the one or more image sequences fail to simultaneously satisfy C11, C13, and C14, the processing device 110 or the processing module 330 determines that the candidate application scenario is incompatible.

In some embodiments, the processing device 110 or the processing module 330 uses an optimization algorithm to redetermine the constraint threshold (e.g., the proportion threshold or the size threshold) based on historical matching data and second user feedback data. The constraint threshold includes proportion thresholds or the size threshold, and the proportion thresholds include the first proportion threshold and the second proportion threshold.

The historical matching data includes the constraint thresholds in the constraint conditions satisfied by historical image sequences, historical target application scenarios, and historical image sequences corresponding to the historical target application scenarios.

The second user feedback data refers to evaluations made by the user regarding the historical image sequences when viewing or using the images in the historical image sequences in the historical target application scenarios. Merely by way of example, the second user feedback data is "a proportion of images of the target object is too great/too small," "an actual physical size of the target object is too great/too small," "the target application scenario is incorrect," etc.

The optimization algorithm may be a gradient search-based algorithm, a Bayesian optimization algorithm, a genetic algorithm, etc.

In some embodiments, the processing device 110 or the processing module 330 constructs a mapping relationship table based on the historical matching data and the second user feedback data. The mapping relationship table includes a mapping relationship between a plurality of constraint thresholds (the constraint thresholds in constraint conditions of a plurality of historical target application scenarios) and a plurality of second user feedback data.

The processing device 110 or the processing module 330 uses the above mapping relationship table as an input to the optimization algorithm and determines a new constraint threshold through iterative computation. Each iteration uses different constraint thresholds within a constraint threshold range (referring to a range where the proportion threshold or the size threshold falls, e.g., a proportion threshold range of [0.6, 0.8] or a size threshold range of [3 mm, 5 mm]), and calculates an average accuracy rate for each constraint threshold based on the second user feedback data. Herein, when the second user feedback data is "a proportion of images of the target object is too great/too small" or "an actual physical size of the target object is too great/too small," an accuracy rate of the constraint threshold is 50%; when the second user feedback data is "the target application scenario is incorrect," the accuracy rate of the constraint threshold is 0; when the second user feedback data is "null" (representing that the user did not evaluate the historical target application scenario or the historical image sequence), the accuracy rate of the constraint threshold is 100%. The processing device 110 or the processing module 330 may record intermediate data generated during the above calculation process (e.g., the accuracy rate of the constraint threshold acquired in each iteration) for subsequent backtracking or manual intervention.

The processing device 110 or the processing module 330 may automatically update the constraint threshold range based on gradient changes or score improvement directions of calculation results from each iteration, thereby gradually converging to an optimal constraint threshold range. The processing device 110 or the processing module 330 may select any constraint threshold within the optimal constraint threshold range as a new constraint threshold.

In some embodiments of the present disclosure, the optimization algorithm is used to automatically determine the constraint threshold to achieve an automatic adjustment of the constraint conditions, thereby realizing a personalized application scenario matching under different device types and patient features, and improving the accuracy and adaptability of the target application scenario.

In some embodiments, the processing device 110 or the processing module 330 adjusts the constraint threshold based on a credibility score of the at least one target application scenario.

Merely by way of example, if the average of the credibility scores of the at least one target application scenario is less than a first credibility threshold, the processing device 110 or the processing module 330 decreases the constraint threshold (e.g., decreases the proportion threshold and the size threshold).

As another example, if the average of the credibility scores of the at least one target application scenario is greater than a second confidence threshold, the processing device 110 or the processing module 330 increases the constraint threshold (e.g., increases the proportion threshold and the size threshold). The second confidence threshold is greater than the first confidence threshold. More contents on the credibility score and the manner for determining the credibility score may be found in later descriptions.

In some embodiments of the present disclosure, by dynamically adjusting the constraint threshold based on the credibility score, the constraint threshold is tightened (increased) when the confidence is too high, and relaxed (decreased) when the confidence is too low, thereby achieving a self-balancing between matching a sensitivity and a specificity.

In some embodiments, each candidate application scenario further corresponds to at least one preceding constraint condition (also referred to as a second constraint condition). In some embodiments, before matching the one or more image sequences with the constraint conditions (also referred to as the first constraint conditions) of the plurality of candidate application scenarios, the processing device 110 or the processing module 330 first filters, from the plurality of candidate application scenarios, a plurality of candidate application scenarios whose second constraint conditions are satisfied by the one or more image sequences (also referred to as advanced candidate application scenarios), and then determines, from the plurality of advanced candidate application scenarios, at least one candidate application scenario that is suitable for the one or more image sequences as the at least one target application scenario.

In some embodiments, the second constraint condition for each of the plurality of candidate application scenarios includes at least one of the following multiple items (C21 to C25):
C21: Among the images in the set of images, there is at least one image whose modality is a preset modality.
C22: The spacing between slices of each of the one or more image sequences is greater than or equal to a spacing threshold.
C23: The spacing between slices of each of the one or more image sequences is less than the spacing threshold.
C24: A layer thickness of the one or more images in each of the one or more image sequences is greater than or equal to a layer thickness threshold.
C25: A count of the images in each of the one or more image sequences (i.e., a count of slice images of the image sequence) is greater than or equal to a count threshold.

It may be understood that as contents of C22 and C23 are mutually exclusive, the second constraint condition of the same candidate application scenario cannot include both C22 and C23.

In some embodiments, if the one or more image sequences satisfy all the second constraint conditions of the candidate application scenario, the processing device 110 or the processing module 330 determines that the candidate application scenario is suitable for the one or more image sequences, and the candidate application scenario serves as the advanced candidate application scenario. Taking the second constraint conditions of the candidate application scenario including C21, C23, and C25 as an example, the one or more image sequences must satisfy C21, C23, and C25 simultaneously. Then, the processing device 110 or the processing module 330 determines that the candidate application scenario is adapted for the one or more image sequences. If the one or more image sequences fail to satisfy C21, C23, and C25 simultaneously, the processing device 110 or the processing module 330 determines that the candidate application scenario is not adapted for the one or more image sequences.

Merely by way of example, the following three examples for determining the target application scenario are used to illustrate the process of determining the target application scenario.

### Embodiment 1:

The processing device 110 or the processing module 330 matches information of the images in the set of images (including the key information and the auxiliary information of at least a portion of images in each of the one or more image sequences) with second constraint conditions of at least one candidate application scenario to determine at least one successfully matched advanced candidate application scenario from the at least one candidate application scenario including: if the inter-slice spacing information of each of the one or more image sequences is greater than or equal to a first spacing threshold, a layer thickness of the image is greater than or equal to a first layer thickness threshold, a count of the slice images is greater than or equal to a first count threshold, and the set of images includes at least one image whose modality is a first preset modality, then determining that the one or more image sequences match the second constraint conditions of a CT brain perfusion application.

Correspondingly, the processing device 110 or the processing module 330 matches the information of the set of images with the first constraint conditions of each of the at least one advanced candidate application scenario respectively, to determine, in the at least one advanced candidate application scenario, at least one target application scenario that is successfully matched with the information of the set of images, including: if a proportion of images whose anatomical region information is the head region in the set of images is greater than or equal to a first proportion threshold, and among the set of images, there are one or more images whose modality information is "brain perfusion enhancement" and "plain scan," determining that the CT brain perfusion application is a successfully matched target application scenario.

The first spacing threshold, the first layer thickness threshold, the first count threshold, the first preset modality, and the first proportion threshold may be set according to requirements of the CT brain perfusion application. For the CT brain perfusion application, it may be determined, based on the information of the set of images, whether the inter-slice spacing of each of the one or more image sequences is greater than or equal to 3 millimeters (mm), whether the layer thickness is greater than or equal to 3 mm, and whether the count of slice images is greater than or equal to 3 layers. If the inter-slice spacing of each of the one or more image sequences is greater than or equal to 3 millimeters (mm), the layer thickness is greater than or equal to 3 mm, and the count of slice images is greater than or equal to 3 layers, it may be determined that the one or more image sequences match the second constraint conditions of the CT brain perfusion application. Further, if among the set of images, a proportion of the images whose anatomical region information is the head region is greater than or equal to 80%, and among the set of images, there are images whose modality information is "brain perfusion enhancement" and "plain scan," then it is determined that the CT brain perfusion application is the successfully matched the one or more image sequences.

In this way, for the input one or more image sequences, using the information of the at least a portion of images in the one or more image sequences to constrain the inter-slice spacing information, the layer thickness, and the count of slice images ensures a sufficient clarity of the target object in the one or more image sequences. Secondly, using the information of the set of images in the one or more image sequences may enable a morphology of the brain to be clearly presented in the one or more image sequences of the brain perfusion enhancement and the plain scan images and be applied to the CT brain perfusion application, so as to satisfy the requirements of the CT brain perfusion application.

### Embodiment 2:

The processing device 110 or the processing module 330 matches information of the set of images with second constraint conditions of at least one candidate application scenario to determine at least one successfully matched advanced candidate application scenario from the at least one candidate application scenario, including: if the inter-slice spacing of each of the one or more image sequences is less than or equal to a second spacing threshold, the count of slice images in each of the one or more image sequences is greater than or equal to a second count threshold, and the set of images includes at least one image whose modality is a second preset modality, then determining that the one or more image sequences match the second constraint conditions of the CT cardiac application. On this basis, the CT cardiac application becomes the advanced candidate application scenario.

Correspondingly, the processing device 110 or the processing module 330 matches the information of the set of images with the first constraint conditions of each of the at least one advanced candidate application scenario, to determine, from the at least one advanced candidate application scenario, at least one target application scenario that is successfully matched, including: if in the set of images, an object with the greatest proportion is the target object (e.g., the heart), there is at least one image in the set of images in which an actual physical size of the heart is greater than or equal to the first size threshold, and among the set of images, there is an image whose modality information is "vascular/coronary enhancement," then determining that the one or more image sequences match the first constraint condition of the CT cardiac application. On this basis, the CT cardiac application becomes a target application scenario.

The second spacing threshold, the second count threshold, the second preset modality, and the first size threshold may be set according to requirements of the CT cardiac application. For the CT cardiac application, it may first be determined, based on the information of the set of images, whether the inter-slice spacing information of each of the one or more image sequences is less than or equal to 1 mm and whether the count of slice images is greater than or equal to 3. If the inter-slice spacing information of each of the one or more image sequence is less than or equal to 1 mm and the count of slice images is greater than or equal to 3, it may be determined that the one or more image sequences match the second constraint condition of the CT cardiac application. Further, if among the set of images, the anatomical region with the greatest proportion is the chest, the actual physical size of the heart is greater than or equal to 10 cm, and there is an image whose modality information is "vascular/coronary enhancement," then it is determined that the CT cardiac application is a successfully matched target application scenario.

In this way, for the input one or more image sequences, using the information of the at least a portion of images in the one or more image sequences to constrain the spacing between slices and the count of slice images ensures a sufficient clarity of the target object in the one or more image sequences. Besides, using the information of the set of images in the one or more image sequences enables the morphology of the chest to be clearly presented in the one or more image sequences of the vascular/coronary enhancement and be applied to the CT cardiac application, so as to satisfy the requirements of the CT cardiac application.

### Embodiment 3:

The processing device 110 or the processing module 330 matches information of the set of images with second constraint conditions of at least one candidate application scenario to determine at least one successfully matched advanced candidate application scenario from the at least one candidate application scenario, including: if the count of slice images of each of the one or more image sequences is greater than or equal to the third count threshold, and the set of images includes at least one image whose modality is a third preset modality, determining that the one or more image sequences match the second constraint conditions of an MR breast analysis application.

Correspondingly, the processing device 110 or the processing module 330 matches the information of the set of images with the first constraint conditions of the at least one advanced candidate application scenario respectively, to determine, from the at least one advanced candidate application scenario, at least one target application scenario that is successfully matched, including: if among the set of images, there is an image whose anatomical region information is the breast region, and there are the images whose modality information is "enhanced scan" and "plain scan," then determining that the MR breast analysis application is a successfully matched target application scenario.

The third count threshold and the third preset modality may be set according to requirements of the MR breast analysis application. For the MR breast analysis application, it may be determined, based on the information of the set of images, whether the count of layers of each of the one or more image sequences is greater than or equal to 3. If the count of layers of each of the one or more image sequences is greater than or equal to 3, it may be determined that the one or more image sequences matches a second constraint condition of the MR breast analysis application. Further, if among the at set of images, there is an image whose anatomical region information is the breast region, and there are images whose modality information is "enhanced scan" and "plain scan," it is determined that the MR breast analysis application is a successfully matched target application scenario.

In this way, for the input one or more image sequences, using the information of the at least a portion of images in the one or more image sequences to constrain the spacing between slices and the count of slice images ensures the sufficient clarity of the target object in the one or more image sequence. Besides, using the information of the set of images in the one or more image sequences enables the morphology of the breast region to be clearly presented in the one or more image sequences whose modality is an enhanced or plain scan and be applied to the MR breast analysis application, so as to satisfy the requirements of the MR breast analysis application.

It may be understood that, after determining at least one target application scenario, the processing device 110 or the processing module 330 needs to push the at least one target application scenario to a user terminal (e.g., the user terminal 150), so that the user uses the at least one target application scenario.

In some embodiments, before pushing the at least one target application scenario to the user terminal, for each of the at least one target application scenario, the processing device 110 or the processing module 330 further determines the credibility score of the target application scenario. In response to the credibility score being less than a credibility threshold (a third confidence threshold), the processing device 110 or the processing module 330 pushes a notification message to the user terminal. In response to the credibility score being greater than or equal to the third confidence threshold, the processing device 110 or the processing module 330 pushes the target application scenario and the credibility score to the user terminal. The third confidence threshold may be preset. The third confidence threshold is less than the first confidence threshold and the second confidence threshold.

The credibility score reflects a degree of credibility of the target application scenario. A higher value of the credibility score of the target application scenario represents a higher degree of credibility of the target application scenario.

The notification message is used to remind the user that the currently determined at least one target application scenario has a low credibility and the application scenario of the one or more image sequences requires to be manually selected. Forms of the notification message include, but are not limited to, texts, images, voices, and animations. Merely by way of example, the notification message is in a text form with a content "No suitable application scenario found."

In some embodiments, the processing device 110 or the processing module 330 determines the credibility score for each target application scenario based on the following process (S11-S15):
S11: based on a count of matching items of the constraint conditions of the target application scenarios, a scenario matching metric may be determined.

The count of matching items refers to a count of first constraint conditions of the target application scenario that is satisfied by the one or more image sequences. Taking the first constraint conditions of the target application scenario including C11, C13, and C14 as an example, if the one or more image sequences satisfy C11 and C14, the count of matching items for the target application scenario is 2.

The scenario matching index reflects a magnitude of the count of matching items. Merely by way of example, a value of the scenario matching index is equal to the count of matching items, or is proportional to the count of matching items.

S12: based on a confidence of an output of the key information extraction model, a model confidence metric may be determined.

The confidence refers to a degree of confidence of the key information of the image output by the key information extraction model. A greater value of the confidence represents that the key information of the image output by the key information extraction model is more credible. The value of the confidence is within a range of [0, 1].

The model confidence metric reflects a global confidence degree of the key information of the images in the set of images. A greater value of the model confidence metric indicates a higher global confidence degree of the key information of the images in the set of images. The value of the model confidence metric may be determined based on the confidence of the key information of each image in the set of images. Merely by way of example, the value of the model confidence metric is equal to a maximum value or an average value of the confidence of the key information of each image in the set of images.

S13: determining an image quality score of each image in the set of images by processing the image using a quality evaluation model. More descriptions regarding the quality evaluation model and the image quality score may be found in FIG. 4 and its related descriptions.

S14: based on the image quality score of the each image in the set of images, a data validity metric may be determined.

The data validity metric reflects a confidence degree of the key information. A greater value of the model confidence metric indicates a higher confidence degree of the key information of the images in the set of images. The value of the data validity metric may be determined based on the image quality score of each image in the set of images. The value of the data validity metric may be equal to an average value of the image quality scores of each image in the set of images.

S15: based on the scenario matching metric, the model confidence metric, and the data validity metric, the credibility score of the target application scenario may be determined. Merely by way of example, the credibility score is a weighted sum of the scenario matching index, the model confidence metric, and the data validity metric, and weights of the scenario matching index, the model confidence metric, and the data validity metric are preset.

In some embodiments, before performing the weighted summation on the scenario matching index, the model confidence metric, and the data validity metric, the scenario matching index, the model confidence metric, and the data validity metric are normalized, and then the weighted sum is determined based on the normalized scenario matching index, the normalized model confidence metric, and the normalized data validity metric.

In some embodiments of the present disclosure, by determining the credibility score for each target application scenario, untrustworthy target application scenarios are automatically filtered out for the user, ensuring a reliability of the target application scenarios received by the user.

In some embodiments, before pushing at least one target application scenario and the one or more image sequences to the user terminal, the processing device 110 or the processing module 330 further performs a predefined operation on each of the at least one target application scenario. The predefined operation is acquired from an operation database. The operation database is configured in a storage device (e.g., the storage device 130). The operation database stores a plurality of predefined operations.

In some embodiments, the predefined operation performed on the target application scenario includes at least one of the following operations (O1~O5):
O1: an image processing algorithm corresponding to the target application scenario may be used to process the one or more image sequences to acquire an image processing result.

The image processing algorithm includes an image segmentation, a alignment, a localization (e.g., a lesion localization), a measurement (e.g., a lesion volume measurement), a 3D modeling, a spectral analysis, a radionuclide calculation, a 4D dynamic display, a hemodynamics calculation, etc. Correspondingly, the image processing result includes an image segmentation result, a alignment result (e.g., a spatial position transformation matrix), a localization result (e.g., coordinates of a lesion), a measurement result (e.g., a lesion volume), a 3D model of the lesion (e.g., a tumor), etc.

O2: a display parameter of a display interface of a user terminal may be configured, and the image processing result may be displayed on the display interface of the user terminal according to the display parameter.

The display parameter includes a display mode (e.g., single-screen display, dual-screen display, or multi-screen display), a display position (a position of the image processing result on the display interface of the user terminal), a display size (an area size of the image processing result on the display interface of the user terminal), or the like, or a combination thereof.

O3: a notification message may be send to the user terminal.

The notification message is configured to remind a user that the one or more image sequences and the target application scenario are acquired, thereby reminding the user to view and process them timely. The notification message may be a voice notification, a pop-up notification (e.g., a text pop-up or an image pop-up), etc.

O4: a control instruction may be send to an imaging device (e.g., the imaging device 140).

The control instruction is configured to adjust one or more reconstruction parameters (e.g., layer thickness and reconstruction spacing, a reconstruction algorithm, a kernel function, a reconstruction field of view, etc.) and/or one or more scan parameters (e.g., a scan modality, a scan protocol, whether to perform a supplementary scan, etc.) of the imaging device.

O5: an information summary report may be generated and send to the user terminal.

The information summary report includes basic information of the imaging object (e.g., a gender, an age, a height, a weight, etc. of the imaging object) and/or the key information of each image, etc.

In some embodiments of the present disclosure, performing the predefined operation after determining the target application scenario achieves an automatic linkage from application scenario identification to clinical operation, thereby improving integration and efficiency of a clinical workflow.

In some embodiments of the present disclosure, performing a comprehensive analysis on the key information and the auxiliary information achieves an automatic determination of an adapted application scenario for the images (e.g., the one or more image sequences), thereby achieving an automated connection from image identification to clinical application matching, reducing user operations, and improving user work efficiency.

In some embodiments, for each image sequence in the one or more image sequences, the processing device 110 or the processing module 330, based on the key information of the images in the image sequence, determines a processing strategy for each image in the image sequence, and processes each image in the image sequence based on the processing strategy for the image. More descriptions regarding the above embodiments may be found in FIG. 11 and the related descriptions.

FIG. 7 is a flowchart illustrating an exemplary training process of a key information extraction model according to some embodiments of the present disclosure. As shown in FIG. 7, process 700 includes the following operations. In some embodiments, process 700 is performed by the processing device 110 or the determination module 320.

In 710, a plurality of sample image sets are acquired.

The sample image set includes a plurality of second sample images and a plurality of sample image sequences. Each sample image sequence includes a plurality of third sample images.

The sample image set may be determined based on historical image sequences acquired from historical scan processes. Merely by way of example, image sequences acquired from historical scan processes are stored in a storage device (e.g., the storage device 130). The processing device 110 or the determination module 320 retrieves a plurality of historical image sequences (e.g., of a same imaging object) from the storage device as the plurality of sample image sequences, and extracts a plurality of historical images from the plurality of historical image sequences as the plurality of second sample images.

It should be noted that the second sample images are extracted from the historical image sequences, and the sample image sequences are directly determined based on the historical image sequences. Therefore, the second sample images may be duplicate images of the third sample images.

A label of the second sample image or the third sample image includes key information corresponding to the image. In some embodiments, after acquiring the plurality of second sample images and the plurality of sample image sequences, the label may be annotated (e.g., manually annotated) for each second sample image and each third sample image to precisely indicate the key information (e.g., anatomical region information or organ information) of the second sample image or the third sample image.

In 720, an initial machine learning model is trained based on the plurality of second sample images to acquire an intermediate model.

Merely by way of example, the processing device 110 or the determination module 320 inputs the plurality of second sample images into an initial key information extraction model, determines a value of a fourth loss function based on an output of the initial key information extraction model and labels of the second sample images, and iteratively trains the initial key information extraction model until a preset condition is satisfied (e.g., the value of the fourth loss function is less than a fourth loss threshold, or an iteration count is greater than or equal to a fourth preset count), thereby acquiring a trained intermediate model.

In some embodiments, the processing device 110 or the determination module 320 uses the plurality of second sample images to pre-train the initial machine learning model to initialize weights of the initial machine learning model, thereby acquiring the intermediate model. Thus, the intermediate model may inherit rich features learned during the pre-training stage, thereby achieving a faster convergence when processing images in the image sequences and reducing a reliance on large-scale annotated data.

In 730, the plurality of sample image sequences are processed based on the intermediate model to acquire prediction information for each third sample image in each of the plurality of sample image sequences.

The prediction information refers to the key information (e.g., the anatomical region information or the organ information) of each third sample image output by the intermediate model after processing the plurality of sample image sequences, and a confidence of the key information.

In 740, the intermediate model is trained based on the prediction information of the plurality of third sample images in the plurality of sample image sequences and labels of the plurality of third sample images in the plurality of sample image sequences to acquire the key information extraction model.

In some embodiments, the processing device 110 or the determination module 320 inputs the plurality of third sample images of the plurality of sample image sequences into the intermediate model, respectively. Based on the prediction information output by the intermediate model and the labels of the third sample images, a value of a fifth loss function is determined. The intermediate model is iteratively trained until a preset condition is satisfied (e.g., a value of the fifth loss function is less than a third loss threshold, or a count of iterations is greater than or equal to a fifth preset count) to acquire the trained key information extraction model.

In some embodiments, during the training of the intermediate model, a corresponding cross-entropy loss function is configured for each classification head. A sum of loss values of all classification heads is used as an overall loss value of the entire intermediate model to comprehensively evaluate a performance of the intermediate model on a classification result of each classification head.

In some embodiments, when processing third sample images involving different anatomical regions, a count of third sample images containing certain anatomical regions far exceeds a count of third sample images containing other anatomical regions. To alleviate this imbalance issue, a random sampling strategy based on the anatomical region may be employed to select the third sample images ultimately used.

In some embodiments, during the training of the intermediate model, parameters of the intermediate model are updated using a gradient descent method based on an Adam optimizer. For example, an initial learning rate of 1e⁻⁵ is set for the Adam optimizer, and after every 50 training periods, a current learning rate is multiplied by 0.5.

In some embodiments, a training batch for the intermediate model is 24, and a count of the training periods is 500. The training batch refers to a count of third sample images used in a single training period.

It should be noted that the aforementioned parameters (e.g., the initial learning rate, a batch size, the count of training periods) are all intended to acquire the key information extraction model with an excellent performance. These parameters are specifically adjusted according to actual dataset features and a model structure to achieve an optimization of the key information extraction model.

In some embodiments, to enhance a generalization capability of the acquired key information extraction model and prevent an overfitting during the training process, before training the initial key information extraction model or the intermediate model, the processing device 110 or the determination module 320 performs a data augmentation on each of the plurality of second sample images or each third sample image in the sample image set. The data augmentation includes, but is not limited to:
Brightness and contrast adjustment: randomly adjusting brightness and contrast of the second sample images or the third sample images to adapt to visual variations caused by differences in equipment and lighting conditions, which helps the key information extraction model recognize subtle features in images (e.g., the first images).

Image sharpening and blurring: enhancing edge information through sharpening to optimize boundary and detail recognition. Simultaneously, applying Gaussian blur and motion blur to simulate blurring effects during image transmission or processing, enhancing an ability of the key information extraction model to process images with different levels of clarity.

Noise addition and downsampling: Adding Gaussian noise to the second sample images or the third sample images to simulate a noise interference during image acquisition, improving a noise resistance of the key information extraction model. Reducing image resolution through downsampling enables the key information extraction model to perform effective recognition under resource-constrained conditions or with poor image quality.

Affine and elastic deformation simulation: Applying affine transformations (e.g., scaling, shearing, translation, etc.) and random elastic deformations to the second sample images or the third sample images to simulate various deformation and displacement situations that occur in the images, thereby enhancing an adaptability of the key information extraction model to complex scenarios.

FIG. 8 is a flowchart illustrating an exemplary method for determining key information of a second image according to some embodiments of the present disclosure. As shown in FIG. 8, a process 800 includes the following operations. In some embodiments, the process 800 is performed by the processing device 110 or the determination module 320.

In 810, a matching result between the second image and each of at least a portion of the one or more first images in a first image sequence is determined. Operation 810 is performed by the processing device 110 or the determination module 320 for each second image in each second image sequence.

In some embodiments, the processing device 110 or the determination module 320 determines the matching result between a second position of the second image and a first position of each of the at least a portion of the one or more first images in the first image sequence.

The first position is a coordinate position of an imaging part of an imaging object corresponding to the first image in a spatial coordinate system. The second position is a coordinate position of an imaging part of the imaging object corresponding to a second image in the spatial coordinate system. The imaging part of the imaging object refers to an actual part of the imaging object corresponding to a reference point of the first image or the second image. the reference point may be a preset point (e.g., a center point or a point in the upper left corner, etc.) in the first image or the second image.

The spatial coordinate system is a 3D coordinate system for calibrating different regions of the imaging object. A coordinate origin of the spatial coordinate system is located within the imaging object. For example, the coordinate origin of the spatial coordinate system is located at a geometric center or a center of gravity of the imaging object when lying flat.

The matching result indicates whether the second position is consistent with the first position.

In some embodiments, the processing device 110 or the determination module 320 determines the matching result between the second position of the second image and the first position of each first image through various manners.

In some embodiments, in response to determining that a distance between the first position and the second position (a coordinate distance in the spatial coordinate system) is less than a distance threshold, the processing device 110 or the determination module 320 determines that the matching result between the first position of the first image and the second position of the second image is consistent. The distance threshold may be preset.

In some embodiments, for each of the one or more second image sequences, the processing device 110 or the determination module 320 performs alignment on the first image sequence and the second image sequence to determine a spatial position transformation matrix. Merely by way of example, the alignment is a rigid alignment, an affine alignment, etc.

It is understandable that after aligning the first image and the second image, a spatial position transformation relationship between the first image and the second image is acquired. The spatial position transformation relationship may include a rotation angle, a rotation direction, a translation distance, etc.

The spatial position transformation matrix includes a plurality of elements. Each element may represent a spatial position transformation relationship between one second image in the second image sequence and one first image in the first image sequence. For example, an element a25 of a spatial position transformation matrix A (an element located in a second row and a fifth column of the spatial position transformation matrix A) indicates a spatial position transformation relationship between the second first image in the first image sequence and the fifth second image in the second image sequence. As another example, an element a43 of the spatial position transformation matrix A (an element located in a fourth row and a third column of the spatial position transformation matrix A) indicates a spatial position transformation relationship between the fourth first image in the first image sequence and the third second image in the second image sequence.

In some embodiments, the processing device 110 or the determination module 320 acquires a plurality of first feature points from the first image sequence and acquires a plurality of second feature points corresponding to the first feature points from the second image sequence. In some embodiments, as incorrect matches occur during the feature point matching process, the processing device 110 or the determination module 320 eliminates mismatched feature points among the first feature points and the second feature points based on manners such as a ratio test or a geometric consistency check to acquire matched first target feature points and second target feature points (i.e., feature point pairs). The processing device 110 or the determination module 320 aligns the first target feature points and the second target feature points by performing a rigid body transformation (including rotation and translation) or an affine transformation (including scaling, rotation, and translation) to acquire the spatial position transformation matrix.

In some embodiments, after determining the spatial position transformation matrix, the processing device 110 or the determination module 320 determines a transformed position of the second image by applying the spatial position transformation matrix on the second position of the second image.

The transformed position refers to a position of the second image after performing a position transformation on the second position of the second image according to the corresponding spatial position transformation relationship in the spatial position transformation matrix. For example, the processing device 110 or the determination module 320 performs rotation and translation processing on the second position of the second image based on the spatial position transformation matrix to acquire the transformed position of the second image, i.e., to acquire the corresponding position of the second image in the first image sequence.

In some embodiments, after determining the transformed position, the processing device 110 or the determination module 320 determines the matching result based on the first position of each first image in the first image sequence and the transformed position of the second image. For example, if the transformed position of the second image is consistent with the first position of the first image, then the second position of the second image is consistent with the first position of the first image, i.e., the second position of the second image matches the first position of the first image. Conversely, if the transformed position of the second image is inconsistent with the first position of the first image, then the second position of the second image is inconsistent with the first position of the first image, i.e., the second position of the second image does not match the first position of the first image.

In some embodiments, the processing device 110 or the determination module 320 also determines the matching result between the second position of the second image and the first position of each of the at least a portion of the one or more first images in other manners (e.g., based on a similarity between a segmentation mask of an organ at the second position in the second image and a segmentation mask of an organ at the first position in the first image), which is not limited herein.

In 820, the key information of the second image is determined based on the matching result and the key information of each of the at least a portion of the one or more first images in the first image sequence. Operation 820 is performed by the processing device 110 or the determination module 320 for each second image in each second image sequence.

In some embodiments, in response to the matching result being that the second position of the second image is consistent with the first position of a first image, determining the key information of the first image corresponding to the matching result as the key information of the second image corresponding to the matching result.

In some embodiments, in response to the matching result being that the second position of the second image is not consistent with the first position of any first image, determining the key information of the second image by processing the second image through the key information extraction model. More descriptions regarding the key information extraction model may be found in FIG. 4 or FIG. 5 and the related descriptions.

In some embodiments, in response to the matching result being that the second position of the second image is not consistent with the first position of any first image, the processing device 110 or the determination module 320 determines an axial depth of the second image in the second image sequence. The axial depth may be determined based on an AFOV and inter-slice spacing information of the image sequence.

Merely by way of example, taking the AFOV of the second image sequence being 15 cm and the inter-slice spacing information being 3 cm as an example, the second image sequence includes a total of six second images. The axial depths of these six second images are 0, +3 cm, +6 cm, +9 cm, +12 cm, and +15 cm, respectively.

In some embodiments, the processing device 110 or the first determination module 320 determines, from the first image sequence, at least one candidate first image whose axial depth is close to the axial depth of the second image (the axial depth of the second image in the second image sequence). The term "close" means that a difference between the axial depth of the candidate first image (the axial depth of the candidate first image in the first image sequence) and the axial depth of the second image is not greater than a difference threshold.

Merely by way of example, taking the axial depth of the second image in the second image sequence being +3 cm and the difference threshold being 1 cm as an example, assuming that the first image sequence includes a total of five first images and the axial depths of these five first images are 0, +2 cm, +4 cm, +6 cm, and +8 cm, respectively, then the first images whose differences are not greater than the difference threshold include the second first image (axial depth of +2 cm) and the third first image (axial depth of +4 cm). These two first images become the candidate first images.

In some embodiments, the processing device 110 or the determination module 320 determines an approximate image of the candidate first image based on the candidate first image. For example, the approximate image corresponding to the candidate first image is determined by performing an interpolation between the candidate first images. Then, the processing device 110 or the first determination module 320 may process the approximate image using the key information extraction model and take the key information output by the key information extraction model as the key information of the second image.

In some embodiments of the present disclosure, compared to directly using the key information extraction model to determine the key information of each second image, reusing the key information of the first image through spatial position matching to indirectly determine the key information of the second image avoids repeatedly invoking the key information extraction model for unnecessary data processing. This significantly reduces a consumption of computing resources and improves an efficiency of determining key information for multiple sequences.

FIG. 9 is a flowchart illustrating an exemplary method for determining one or more target image sequences according to some embodiments of the present disclosure. As shown in FIG. 9, process 900 includes the following operations. In some embodiments, process 900 is performed by the processing device 110 or the processing module 330.

In 910, a sequence label for each of the one or more image sequences is determined based on the key information of at least a portion of the one or more images in each of the one or more image sequences.

The sequence label is a semantic identifier that reflects image labels of the at least a portion of the one or more images in the image sequence. The sequence label is used to describe an imaging anatomical region range of an imaging object, a modality feature, a disease diagnosis result, etc. corresponding to the image sequence. The sequence label may include an inter-slice spacing, a count of slice images, frequencies or proportions of various types of anatomical region information in the image sequence (frequencies or proportions of various types of anatomical region information among the anatomical region information of all images in the image sequence), frequencies or proportions of various types of organ information (frequencies or proportions of various types of organ information among the organ information of all images in the image sequence), frequencies or proportions of various types of modality information (frequencies or proportions of various types of modality information among the modality information of all images in the image sequence), etc.

The image label refers to a textual description of the key information of an image, and is used to express semantic attributes of the image. Taking the image label of a certain image being "chest-lung-T1" as an example, the image label indicates that the anatomical region information in the key information of the image is the chest region, the organ information is the lung, and the modality information is a T1-weighted imaging. More descriptions regarding the key information, the anatomical region information, organ information, and the modality information may be found in FIG. 4 and the related descriptions.

In some embodiments, the processing device 110 or the processing module 330 statistically determines a frequency of occurrence of various key information based on the key information of the at least a portion of the one or more images in the image sequence. The processing device 110 or the processing module 330 determines the sequence label of the image sequence based on the frequency of occurrence of the various key information.

Merely by way of example, if a frequency of occurrence of a type of key information among the key information of the at least a portion of the one or more images in the image sequence is greater than a frequency threshold, the sequence label of the image sequence includes the type of key information. The frequency threshold may be preset. For example, the frequency threshold may be preset as 8, 10, 15, etc.

In some embodiments, the processing device 110 or the processing module 330 statistically determines a frequency of occurrence of various key information based on the key information of the at least a portion of the one or more images in the image sequence. The processing device 110 or the processing module 330 determines proportions of the various key information based on the frequency of occurrence of the various key information and a count of images in the image sequence. The processing device 110 or the processing module 330 determines the sequence label of the image sequence based on the proportions of the various key information.

Taking the key information being the anatomical region information as an example, assuming that the at least a portion of the one or more images in the image sequence includes 10 images in total, and the anatomical region information of 5 images includes a neck region, the anatomical region information of 3 images is an abdomen region, and the anatomical region information of 2 images is a chest region, then the frequency of occurrence of the neck region is 5, the frequency of occurrence of the abdomen region is 3, and the frequency of occurrence of the chest region is 2. Herein, the proportion of the neck region is 5/10 × 100% = 50%, the proportion of the abdomen region is 3/10 × 100% = 30%, and the proportion of the chest region is 2/10 × 100% = 20%. Further, the proportions of various anatomical region information in the sequence label are "neck region -50%, abdomen region -30%, chest region -20%."

The manner of determining the proportions of various organ information, the proportions of various modality information, or proportions of other key information (e.g., disease information, image quality information) in the sequence label is the same as the manner of determining the proportions of various anatomical region information described above, which is not repeated here.

In some embodiments of the present disclosure, determining the sequence label of the image sequence based on the proportions of various the key information improves an accuracy of the sequence label of the image sequence and achieves a transition from the semantic annotation of individual images to an automatic summarization of the overall sequence semantics, which helps to improve a subsequent analysis efficiency.

In 920, a search label is determined.

The search label refers to a feature label generated based on a first acquisition instruction, used to determine whether the sequence label of each of the one or more image sequences satisfies at least one search condition. The search label is composed of at least one search condition. The search condition may be used to describe conditions that various data in the sequence label of a searched image sequence (e.g., the proportions of various anatomical region information in the sequence label, the proportions of various organ information in the sequence label, etc.) needs to satisfy. Merely by way of example, the search label is "a count of slice images is greater than 20; and among the anatomical region information of all images in a searched image sequence, the proportion of 'neck region ' is greater than 50%, and the proportion of 'head region ' is greater than 20%." As another example, the search label is "an AFOV of a searched image sequence is greater than 15 cm; and among the disease information of all images in a searched image sequence, the proportion of 'tumor present' is greater than 40%."

The first acquisition instruction refers to an instruction for retrieving the one or more target image sequences. The first acquisition instruction is generated by user demand input by the user. The user demand reflects a type of key information corresponding to images in the one or more target image sequences.

Merely by way of example, taking the user demand being "one or more image sequences corresponding to the heart" as an example, the processing device 110 or the processing module 330 may be generate a search label as "the proportion of 'heart' in various organ information is greater than a third proportion threshold". The third proportion threshold may be preset. As an example, the third proportion threshold may be preset as 50%, 60%, or 70%, etc.

In 930, one or more target image sequences are determined from the one or more image sequences based on the search label and the sequence label of each of the one or more image sequences.

In some embodiments, the processing device 110 or the processing module 330 determines a matching degree between the sequence label of each of the image sequences and the search label, and determines the one or more target image sequences based on the matching degree between the sequence label and the search label of each of the image sequences.

The matching degree between the sequence label of an image sequence and the search label reflects a similarity degree between the sequence label of the image sequence and the search label. A greater value of the matching degree indicates a higher similarity degree between the sequence label of the image sequence and the search label.

The matching degree may be represented by a proportion of the search conditions satisfied by the sequence label of the image sequence among all search conditions of the search label.

Merely by way of example, taking the search label being "the count of slice images is greater than 20; the proportion of 'neck region' in the anatomical region information of all images in a searched image sequence is greater than 50%, the proportion of 'head region' is greater than 20%; the proportion of 'tumor present' in the disease information of all images in a searched image sequence is greater than 40%; the proportion of 'CT plain scan' in the modality information of all images in this image sequence is greater than 90%" as an example, the search label includes five search conditions in total. If the sequence label of the image sequence satisfies all five search conditions above, the matching degree between the image sequence and the search label is 1. If the sequence label of he image sequence satisfies four of the five search conditions above, the matching degree between the image sequence and the search label is 4/5=0.8. If the sequence label of the image sequence satisfies three of the five search conditions above, the matching degree between the image sequence and the search label is 3/5=0.6, and so on, which is not repeated here.

In some embodiments, the processing device 110 or the processing module 330 determines an image sequence, among the one or more image sequences, with a maximum matching degree (e.g.,1) as the target image sequence.

In some embodiments, the processing device 110 or the processing module 330 determines the image sequence with a matching degree greater than or equal to a matching degree threshold as the target image sequence. The matching degree threshold may be preset. For example, the matching degree threshold is preset to 0.7, 0.8, 0.9, etc.

In some embodiments, the processing device 110 or the processing module 330 sorts the one or more image sequences according to the matching degrees (e.g., in a descending order) to acquire a matching degree sorting result, and determines one or more (n count of) image sequences at a top of the matching degree sorting result as the one or more target image sequences. Here, n is a positive integer, n is greater than or equal to 1, and n is less than the count of one or more image sequences.

In some embodiments, when performing the sorting above, the processing device 110 or the processing module 330 may further sort image sequences with the same matching degree based on sequence labels of the image sequences with the same matching degree. For example, the processing device 110 or the processing module 330 sorts each image sequence with the same matching degree according to the proportion of "tumor present" in the disease information.

In some embodiments of the present disclosure, determining one or more target image sequences based on the key information or the sequence label achieves an automatic retrieval of the one or more target image sequences, avoids manual search operations, and improves the efficiency and accuracy of an overall process of image invocation and display.

FIG. 10 is another flowchart illustrating an exemplary method for determining one or more target image sequences according to some embodiments of the present disclosure. As shown in FIG. 10, process 1000 includes the following operations. In some embodiments, process 1000 is performed by the processing device 110 or the processing module 330.

In 1010, a sequence label for each of the one or more image sequences is determined based on the key information of at least a portion of the one or more images in each of the one or more image sequences. More descriptions regarding the sequence label may be found in FIG. 4 or FIG. 9 and the related descriptions.

In 1020, a first target image sequence is determined from the one or more image sequences.

The first target image sequence is one of the one or more image sequences. The first target image sequence is an image sequence that the user (e.g., the physician) wants to retrieve, view, or process. As mentioned previously, each image sequence of the one or more image sequences includes images, and the first target image sequence is one of the one or more image sequences. Therefore, the first target image sequence includes one or more images.

In some embodiments, operation 430 is performed by the processing device 110 or the processing module 330 in response to a second acquisition instruction for the first target image sequence, and the first target image sequence is determined based on the second acquisition instruction.

The second acquisition instruction refers to an instruction input by the user for retrieving the first target image sequence. The second acquisition instruction includes an identifier (e.g., a name or a count) of the first target image sequence. Merely by way of example, a user inputs the second acquisition instruction for the first target image sequence via a user terminal (e.g., the user terminal 150). The instruction is transmitted to a storage device (e.g., the storage device 130) storing the image sequences. Then, the storage device, in response to receiving the second acquisition instruction, sends the first target image sequence to the user terminal for the user to view.

In 1030, at least one second target image sequence is determined from the one or more image sequences.

A second target image sequence refers to an image sequence that has a certain correlation with the first target image sequence. Merely by way of example, the correlation is that the imaging object corresponding to the second target image sequence is the same as the imaging object corresponding to the first target image sequence. As another example, the correlation is that the anatomical region or the organ of the imaging object in the second target image sequence is at least partially the same as the anatomical region or the organ of the imaging object corresponding to the first target image sequence. As another example, the correlation is that the anatomical region or the organ of the imaging object in the second target image sequence and the anatomical region or the organ of the imaging object corresponding to the first target image sequence are located within a same spatial region. Merely by way of example, the anatomical region or the organ of the imaging object in the second target image sequence is adjacent to the anatomical region or the organ of the imaging object in the first target image sequence. In some embodiments, the first target image sequence and the at least one second target image sequence are corresponding to a same anatomical region or a same organ.

In some embodiments, the first target image sequence and the at least one second target image sequence are corresponding to a same anatomical region or a same organ.

In some embodiments, among other image sequences besides the first target image sequence, if the key information of the one or more images in the image sequence is completely identical to the key information of the one or more images in the first target image sequence, the processing device 110 or the processing module 330 determines the image sequence as the second target image sequence. Merely by way of example, assuming that the key information of the images in the first target image sequence is "abdomen-liver-presence of tumor," the processing device 110 or the processing module 330 retrieves, from the one or more image sequences, all image sequences containing images with the key information "abdomen-liver-presence of tumor" as the second target image sequences.

In some embodiments, among other image sequences besides the first target image sequence, if the key information of the one or more images in the image sequence is partially identical to the key information of the one or more images in the first target image sequence, the processing device 110 or the processing module 330 determines the image sequence as the second target image sequence. Merely by way of example, assuming key information of at least one image in the first target image sequence is "abdomen-liver-presence of tumor," the processing device 110 or the processing module 330 may, from the one or more image sequences, retrieve all image sequences containing at least one image with key information including "abdomen" or "liver" or "presence of tumor" as second target image sequences.

In some embodiments, among other image sequences besides the first target image sequence, if the key information of at least one image in the image sequence corresponds to the anatomical region or the organ adjacent to the anatomical region or organ corresponding to the key information of at least one image in the first target image sequence, the processing device 110 or the processing module 330 determines the image sequence as the second target image sequence. Merely by way of example, assuming that the organ information of at least one image in the first target image sequence is "stomach," the processing device 110 or the processing module 330 retrieves, from the one or more image sequences, all image sequences containing at least one image with the organ information being "liver" or "pancreas" or "spleen" (these three organs are all adjacent to the stomach) as the second target image sequences.

In some embodiments of the present disclosure, determining at least one second target image sequence based on the key information or the sequence label achieves an automatic retrieval of the second target image sequence, avoids manual search operations, and improves the efficiency and accuracy of an overall process of image invocation and display.

FIG. 11 is another flowchart illustrating an exemplary process for image processing according to some embodiments of the present disclosure. As shown in FIG. 11, a process 1100 includes the following operations. In some embodiments, the process 1100 is executed by the processing device 110 or the processing module 330.

In 1110, for each of the one or more image sequences, based on the key information of each image in at least a portion of the one or more images in the image sequence, a processing strategy for the image is determined.

In some embodiments, for each image in the at least a portion of the one or more images, the processing device 110 or the processing module 330 processes the image using the key information extraction model to determine the key information of the image. In this case, an input of the key information extraction model includes an image in the at least a portion of the one or more images, and an output includes the key information of the image. More descriptions of the key information extraction model may be found in FIG. 4, FIG. 5, FIG. 7, and related descriptions thereof.

In some embodiments, the feature information of each of the one or more image sequences further includes additional information corresponding to each of the one or more image sequences.

The additional information refers to non-image information related to each of the one or more image sequences.

In some embodiments, the additional information includes at least one of text information, voice message information, and video information.

Merely by way of example, the text information includes basic information of the imaging object (e.g., a gender, an age, a height, a weight, etc. of the imaging object), a diagnostic report, a content of an examination list, a content of a doctor's advice, etc. The voice message information includes voice describing basic information of the imaging object, a diagnostic report, a content of an examination list, a content of a doctor's advice, etc. The video information includes a video showing a lesion structure of an imaging object.

The additional information may be extracted from a treatment document (e.g., a doctor's advice document, a record document) corresponding to the imaging object of each of the one or more image sequences and standard public tags defined in a DICOM specification.

In some embodiments, the processing device 110 or the processing module 330 extracts an additional feature of the additional information of each image in the at least a portion of the one or more images in each of the one or more image sequences. for each image in the at least a portion of the one or more images in each of in the image sequence, the processing device 110 or the processing module 330 extracts an image feature of the image, and determines the key information by fusing the image feature and the additional feature. In some embodiments, the processing device 110 or the processing module 330 execute the above operations by using an artificial intelligence model.

In some embodiments, the artificial intelligence model includes an image feature extraction model and an additional feature extraction model.

In some embodiments, the processing device 110 or the processing module 330 processes the additional information using the additional feature extraction model to extract the additional feature of the additional information.

The additional feature extraction model refers to a pre-trained natural language processing model based on a transformer architecture. An input of the additional feature extraction model includes the additional information, and an output includes the additional feature of the additional information. For example, the additional feature extraction model is a BERT-type language model.

The additional feature refers to a structured, numerical semantic encoding representation of the additional information. For example, the additional feature may be the text information in a vector format.

In some embodiments, for each image in the at least a portion of the one or more images in each of the one or more image sequences, the processing device 110 or the processing module 330 processes the image using the image feature extraction model to extract the image feature of the image.

The image feature reflects features corresponding to the image. For example, the image feature includes a geometric feature (e.g., a contour or a shape) of anatomical regions or organs contained in the image and an intensity-based feature (e.g., an average or a standard deviation of grayscale values for all pixels in the Image), etc.

In some embodiments, the processing device 110 or the processing module 330 determines the key information by fusing the image feature and the additional feature via a cross-modal transformer.

The cross-modal Transformer is constructed based on a cross-modal attention mechanism. This cross-modal attention mechanism allows the image feature and the additional feature to query each other within a same semantic space. Specifically, the cross-modal attention mechanism allows the image feature to serve as a query to retrieve key semantic information from the additional feature, while the additional feature also serves as a query to search for relevant visual patterns in the image, thereby achieving precise feature alignment and information enhancement. For example, a semantic meaning of a term "pulmonary nodule" in the additional feature guides the attention mechanism to focus on a specific subtle structure in a lung region of the image feature. Conversely, a specific anatomical pattern displayed in the image reinforces or verifies related contents in the additional feature.

The anatomical region information and the additional feature are fused via a classifier decoder to acquire the key information. For example, the key information includes a fusion result of anatomical region information (e.g., "lung") and disease information (e.g., "presence of pulmonary nodule") (e.g., "presence of pulmonary nodule in the lung"). In some embodiments, the cross-modal transformer also outputs a confidence of the key information.

In some embodiments of the present disclosure, by deeply fusing the anatomical region information extracted from the image and the semantic feature (the additional feature) extracted from additional data via cross-modal Transformer technology, the finally determined key information possesses both visual accuracy and contextual relevance.

In some embodiments, a sequence type of each of the one or more image sequences is determined according to DICOM information of the image sequence, and a differentiated processing strategy is adopted for the sequence type.

The DICOM information includes metadata of each image in the one or more image sequences as described above. More descriptions of the metadata may be found in FIG. 4 and related descriptions thereof.

The sequence type reflects a type of images in the image sequence. The sequence type includes a thick-layer 3D sequence, a thin-layer 3D sequence, a multi-time sequence, etc.

A 2D sequence refers to an image sequence where a layer thickness of the images in the image sequence is less than a first thickness threshold.

The thick-layer 3D sequence refers to an image sequence where a layer thickness of the images in the image sequence is greater than a second thickness threshold.

The thin-layer 3D sequence refers to an image sequence where the layer thickness of the images in the image sequence is greater than the first thickness threshold and less than a third thickness threshold. Among the three thickness thresholds, the first thickness threshold is the smallest, the second thickness threshold is the largest, and the third thickness threshold is greater than the first thickness threshold and less than the second thickness threshold.

More descriptions of the multi-time sequence and the layer thickness may be found in FIG. 4 and related descriptions thereof.

A differentiated determination strategy refers to different key information determination strategies adopted for image sequences of different sequence types.

For example, if the sequence type of an image sequence is the 2D sequence, the determination strategy adopted by the processing device 110 or the processing module 330 is: directly inputting any image in the image sequence into the key information extraction model to acquire the key information output by the key information extraction model.

As another example, if the sequence type of an image sequence is the thick-layer 3D sequence, the determination strategy adopted by the processing device 110 or the processing module 330 is: for each of the at least a portion of the one or more images in the in the image sequence (referred to as a to-be-processed image), splitting sub-scan data corresponding to the to-be-processed image into a plurality of 3D data blocks (e.g., splitting the sub-scan data with a thickness of 2 cm into four 3D data blocks each with a thickness of 5 mm), selecting at least one 3D data block from the plurality of 3D data blocks (e.g., arbitrarily selecting two from the four 3D data blocks) and fusing the at least one 3D data block to acquire a fused data block, and then reconstructing a first reference image based on the fused data block. The first reference image is processed by using the key information extraction model to determine the key information of the first reference image, and the key information of the first reference image output by the key information extraction model is used as the key information of the to-be-processed image.

As another example, if the sequence type of an image sequence is the thin-layer 3D sequence, the determination strategy adopted by the processing device 110 or the processing module 330 is: for each to-be-processed image in the image sequence, down-sampling the sub-scan data corresponding to the to-be-processed image, and reconstructing based on the down-sampled sub-scan data to acquire the second reference image. The key information extraction model is used to process the second reference image to determine key information of the second reference image, and the key information of the second reference image output by the key information extraction model is used as the key information of the to-be-processed image.

As another example, if the sequence type of an image sequence is the multi-time sequence, the determination strategy adopted by the processing device 110 or the processing module 330 is: selecting a portion of the images in the image sequence as representative images, and input the representative images into the key information extraction model to determine the key information of the representative images.

In some embodiments, the artificial intelligence model includes a multi-modal large model. The multi-modal large model includes a visual encoder, a additional encoder, and a large language model.

The visual encoder includes a Medical Vision-Text Contrastive Learning (MedCLIP) model and a Vision Transformer Large (ViT-L). The MedCLIP is configured to process a 3D image data block to extract a 3D visual feature (e.g., the anatomical region information). The ViT-L is configured to process the 2D image to extract the visual feature of the 2D image.

The additional encoder includes a model based on Large Language Model Meta AI (LLaMA). The additional encoder is configured to encode the additional information (e.g., the text information) into the additional feature.

The large language model (e.g., GPT) is configured to process a fusion result (e.g., a fusion result acquired through concatenation or an attention mechanism) of outputs of the visual encoder and the additional encoder (i.e., the visual feature and the text information of a vector format) to acquire the key information of the image.

In some embodiments, the multi-modal large model is acquired by inputting the outputs of the visual encoder and the additional encoder into the large language model for pre-training and fine-tuning.

In some embodiments, based on the key information, the processing device 110 or the processing module 330 performs a pre-processing on the at least a portion of the one or more images images in each of the one or more image sequences to acquire a pre-processing result of each image.

The pre-processing here is the second pre-processing. Correspondingly, the second pre-processing result is a second pre-processing result. More descriptions regarding the second pre-processing may be found in FIG. 4 and the related descriptions.

In some embodiments, the processing device 110 or the processing module 330 processes the image and the pre-processing result using the key information extraction model, and uses the key information output by the key information extraction model as updated key information of the image.

More descriptions regarding updating the key information may be found in FIG. 4 and the related descriptions.

The processing strategy includes an operation required to be performed when sending each image in the one or more image sequences to a user terminal. For example, the processing strategy includes pushing an image sequence of an emergency case and the key information of a plurality of images in the image sequence to the user terminal. As another example, the processing strategy includes performing a post-processing on the key information of the images in an image sequence of a normal case first, and then pushing the image sequence of the normal case and the key information of the plurality of images in the image sequence to the user terminal.

In some embodiments, the processing device 110 or the processing module 330 determines the processing strategies for the images in the one or more image sequences based on a strategy correspondence table. The strategy correspondence table records various key information and various pre-processing results, and the processing strategies respectively corresponding to the various key information and the various pre-processing results. The processing device 110 or the processing module 330 may determine the processing strategy corresponding to the image by checking the strategy correspondence table based on the key information and the pre-processing result of the image.

In some embodiments, for each of the one or more image sequences, in response to the key information of at least one image in the image sequence reflecting that an imaging object corresponding to the image sequence is an emergency case, the processing device 110 or the processing module 330 pushes the at least one image and/or the key information of the at least one image to a user terminal.

The emergency case refers to the imaging object suffering from an acute or critical illness. For example, the emergency case is the imaging object suffering from cerebral hemorrhage, cardiac arrest, acute myocardial infarction, etc.

In some embodiments, the processing device 110 or the processing module 330 determines whether the imaging object is the emergency case based on a disease correspondence table. The disease correspondence table records a plurality of diseases and case classification results corresponding to the plurality of diseases. The processing device 110 or the processing module 330 determines the disease of the imaging object based on the disease information of the image, and determines whether the imaging object is the emergency case by checking the disease correspondence table. The case classification result includes an emergency case or an ordinary case (a non-emergency case).

In some embodiments, for each of the one or more image sequences, in response to the key information of none of the images in the image sequence reflecting that the imaging object corresponding to the image sequence is the emergency case, for each image in the image sequence, the processing device 110 or the processing module 330 processes the image through a post-processing algorithm corresponding to the image based on the key information of the image, to obtain a post-processing result and pushes the post-processing result to the user terminal.

The post-processing algorithm includes a finer segmentation, a alignment, a localization (e.g., a lesion localization), a measurement (e.g., a lesion volume measurement), a 3D modeling, a spectral analysis, a radionuclide calculation, a 4D dynamic display, and a hemodynamics calculation, etc.

In some embodiments, the processing device 110 or the processing module 330 determines the post-processing algorithms respectively corresponding to each image in the image sequence based on an algorithm correspondence table. The algorithm correspondence table records various key information and post-processing algorithms respectively corresponding to the various key information. Merely by way of example, for the image whose modality information is "CT enhanced scan" and whose anatomical region information is "chest region," the post-processing algorithm corresponding to the image includes the segmentation, a centerline extraction, a plaque segmentation, etc. As another example, for the image whose anatomical region information is "abdomen region" and whose disease information is "tumor present," the post-processing algorithm corresponding to the image includes a tumor segmentation, a tumor size measurement, etc. The processing device 110 or the processing module 330 may determine a post-processing strategy corresponding to the image by checking the algorithm correspondence table based on the key information of the image.

In some embodiments, the post-processing algorithm corresponding to the image is determined by an intelligent agent. In some embodiments, a process for the intelligent agent to determine the post-processing algorithm includes the following operations S21-S23.

S21: text descriptions of the plurality of post-processing algorithms are registered. The intelligent agent performs a metadata encapsulation on all available post-processing algorithms, thereby generating a structured text description for each post-processing algorithm. A content encapsulated (i.e., the metadata) includes a function name (a unique identifier of the post-processing algorithm), input parameters (e.g., the image sequence, a region of interest, etc.), an operation to be performed by the post-processing algorithm (e.g., the segmentation, the alignment, the measurement, the 3D reconstruction, etc.), a textual functional description of the aforementioned operation (e.g., "for automatic segmentation of lung regions" or "for registration of cardiac time sequence images," etc.), and an applicable scope of the post-processing algorithm (e.g., "applicable to CT chest images" or "for detecting pulmonary nodules"), etc.

S22: a prompt word corresponding to the plurality of post-processing algorithms is generated. The intelligent agent uses a prompt word generation module to fuse the above text descriptions into a structured prompt word. The prompt word generation module may generate the prompt word based on two steps (a template filling and a large model language processing). The template filling refers to acquire a predefined prompt word generation template. An exemplary prompt word generation template may be: "According to the image recognition result: { image result}, selecting the most suitable algorithm from the following algorithm list: {algorithm _list}." The large model language processing refers to using an auxiliary large model (e.g., GPT) to rewrite the image result (e.g., key information) and the algorithm list into a more natural prompt word to improve a decision accuracy.

S23: the prompt word corresponding to the plurality of post-processing algorithms is processed based on the large language model (e.g., the GPT-4 or a medical-specific LLM) to determine a target post-processing algorithm (the target post-processing algorithm refers to the post-processing algorithm determined for subsequent use). S23 further includes the following steps S231-S233.

S231: prompt word input: the prompt word generated in S22 is input into the large language model.

S232: the prompt word is processed using a self-attention mechanism to identify main information of the prompt word (e.g., an image content, an algorithm function, etc.) and a matching degree between the post-processing algorithm and image requirements is calculated. Merely by way of example, the large language model with the self-attention mechanism focuses on "pulmonary nodule" in the prompt word and "lung segmentation" in the algorithm description, thereby establishing an association between the "pulmonary nodule" and the "lung segmentation."

S233: algorithm matching: the large language model determines the target post-processing algorithm from all available post-processing algorithms based on a semantic similarity and a logical reasoning. An output of the large language model is usually a name and a confidence of one or more algorithms. Merely by way of example, the output of the large language model is "Recommend using the pulmonary_nodule_segmentation algorithm, confidence 90%."

In some embodiments of the present disclosure, different processing strategies are intelligently determined according to different images. This not only ensures that the emergency cases receive a prioritized and rapid treatment, thereby guaranteeing a timeliness of diagnosis and treatment for emergency cases, but also automatically matches precise post-processing algorithms for non-emergency cases, thereby optimizing a resource allocation and comprehensively improving a processing efficiency.

In 1120, each image in the image sequence is processed based on the processing strategy for the image.

In some embodiments of the present disclosure, by fusing multimodal patient data (images and texts) and using the artificial intelligence model to perform comprehensive analyses, a more comprehensive and accurate understanding of the image content is achieved, providing a reliable data foundation for subsequent automatic pre-processing and intelligent decision-making.

Finally, it should be understood that the embodiments described in the present disclosure are merely illustrative of the principles of the embodiments of the present disclosure. Other variations may also fall within the scope of the present disclosure. Therefore, by way of example and not limitation, alternative configurations of the embodiments of the present disclosure may be considered consistent with the teachings of the present disclosure. Accordingly, the embodiments of the present disclosure are not limited to the embodiments explicitly introduced and described in the present disclosure.

## Claims

1. A method for image processing, wherein the method comprises:
obtaining one or more image sequences, each of the one or more image sequences including one or more images;
determining key information of at least a portion of the one or more images in each of the one or more image sequences, the key information at least including at least one of anatomical region information, disease information, image quality information, image modality information, an image sequence type, or contrast information; and
processing the one or more image sequences based on feature information of each of the one or more image sequences, the feature information of each of the one or more image sequences including the key information of the at least a portion of the one or more images in the image sequence.

2. The method according to claim 1, wherein the one or more image sequences includes a first image sequence and one or more second image sequences, wherein the first image sequence has a largest axial field of view among the one or more image sequences, the first image sequence includes one or more first images, and each of the one or more second image sequences includes one or more second images, the determining key information of at least a portion of the one or more images in each of the one or more image sequences includes:
determining key information of each of at least a portion of the one or more first images in the first image sequence;
for each of at least a portion of the one or more second images in each of the one or more second image sequences,
determining a matching result between the second image and each of the at least a portion of the one or more first images in the first image sequence; and
determining the key information of the second image based on the matching result and the key information of each of the at least a portion of the one or more first images in the first image sequence.

3. The method according to claim 2, wherein determining a matching result between the second image and the at least a portion of the one or more first images in the first image sequence includes:
determining a matching result between a second position of the second image and a first position of each of the at least a portion of the one or more first images in the first image sequence.

4. The method according to claim 3, wherein the determining the key information of the second image based on the matching result and the key information of each of the at least a portion of the one or more first images in the first image sequence includes:
in response to the matching result being that the second position of the second image is consistent with the first position of the first image, determining the key information of the first image corresponding to the matching result as the key information of the second image corresponding to the matching result;
in response to the matching result being that the second position of the second image is not consistent with the first position of the first image, determining the key information of the second image by processing the second image through a key information extraction model.

5. The method according to any one of claims 1 to 4, wherein the processing the one or more image sequences based on feature information of each of the one or more image sequences includes:
determining one or more target image sequences from the one or more image sequences based on the key information of the at least a portion of the one or more images in each of the one or more image sequences;
sending the one or more target image sequences to a user terminal for display.

6. The method according to claim 5, wherein the feature information of each of the one or more image sequences further includes a sequence label, the determining one or more target image sequences from the one or more image sequences based on the key information of the at least a portion of the one or more images in each of the one or more image sequences includes:
determining the sequence label of each of the one or more image sequences based on the key information of the at least a portion of the one or more images in each of the one or more image sequences;
determining a search label, the search label being used to determine whether the sequence label of each of the one or more image sequences satisfies at least one search condition;
determining the one or more target image sequences from the one or more image sequences based on the search label and the sequence label of each of the one or more image sequences.

7. The method according to claim 6, wherein the determining the sequence label of each of the one or more image sequences based on the key information of the at least a portion of the one or more images in each of the one or more image sequences includes:
determining a frequency of occurrence of various key information based on the key information of the at least a portion of the one or more images in the image sequence;
determining the sequence label of the image sequence based on the frequency of occurrence of the various key information.

8. The method according to any one of claims 1 to 7, wherein the processing the one or more image sequences based on feature information of each of the one or more image sequences includes:
determining at least one target application scenario adapted to the one or more image sequences based on the key information of the at least a portion of images in each of the one or more image sequences.

9. The method according to claim 8, wherein the feature information of each of the one or more image sequences further includes auxiliary information of the at least a portion of the one or more images in each of the one or more image sequences, the auxiliary information including metadata of each image and/or clinical data information of an imaging object corresponding to the image sequence, the determining at least one target application scenario adapted to the one or more image sequences includes:
based on the key information and the auxiliary information of the at least a portion of images in the one or more image sequences, selecting at least one candidate application scenario adapted to the one or more image sequences from a plurality of candidate application scenarios as the at least one target application scenario.

10. The method according to claim 9, wherein the selecting at least one candidate application scenario adapted to the one or more image sequences including:
matching the key information and the auxiliary information of images in a set of images with constraint conditions of the plurality of candidate application scenarios, to determine the at least one candidate application scenario adapted to the one or more image sequences from the plurality of candidate application scenarios as at least one target application scenario, wherein the set of images includes the at least a portion of images in each of the one or more image sequences.

11. The method according to any one of claims 8 to 10, wherein the determining at least one target application scenario adapted to the one or more image sequences further includes:
for each image sequence in the one or more image sequences,
based on the key information of each image in the at least a portion of the one or more images, performing pre-processing on the image sequence to obtain a pre-processing result corresponding to each image in the at least a portion of the one or more images, wherein the pre-processing includes at least one of image segmentation, image alignment, image classification, or artifact removal;
based on the pre-processing result, updating the key information of each image in the at least a portion of the one or more images.

12. The method according to any one of claims 1 to 11, wherein the processing the one or more image sequences based on feature information of each of the one or more image sequences includes:
for each image sequence in the one or more image sequences,
based on the key information of each image in the at least a portion of the one or more images in the image sequence, determining a processing strategy for the image;
processing each image in the at least a portion of the one or more images in the image sequence based on the processing strategy for the image.

13. The method according to claim 12, wherein the feature information of each of the one or more image sequences further includes additional information corresponding to each of the one or more image sequences, the determining key information of at least a portion of the one or more images in each of the one or more image sequences including:
extracting an additional feature of the additional information;
extracting an image feature of the image; and
determining the key information by fusing the image feature and the additional feature.

14. The method according to claim 12 or claim 13, wherein the determining a processing strategy for the image sequence includes:
in response to the key information of at least one image in the image sequence reflecting that an imaging object corresponding to the image sequence is an emergency case, pushing the at least one image and/or the key information of the at least one image to a user terminal for emergency handling by a physician;
in response to the key information of none of the images in the image sequence reflecting that the imaging object is the emergency case, for each image in the image sequence, processing the image through a post-processing algorithm corresponding to the image based on the key information of the image, to obtain a post-processing result and pushing the post-processing result to the user terminal.

15. A computer device, comprising a memory and a processor, wherein the memory stores a computer program, and when the computer program is executed by the processor, the method for image processing of any one of claims 1 to 14 is realized.
